# EUROPÄISCHE PATENTSCHRIFT

(11) **EP 2 566 609 B1**
(45) Veröffentlichungstag und Bekanntmachung des Hinweises auf die Patenterteilung: **29.10.2014**
(21) Anmeldenummer: 11719236.9
(22) Anmeldetag: 06.05.2011
(51) Int. Cl.: B01F 3/08, B01F 7/00, B01F 7/18, B01F 13/08, B01F 13/10, B01F 15/00, B01F 15/06, B01F 15/04

(54) **EMULGIEREINRICHTUNG ZUR KONTINUIERLICHEN HERSTELLUNG VON EMULSIONEN UND/ODER DISPERSIONEN**
EMULSIFICATION DEVICE FOR CONTINUOUSLY PRODUCING EMULSIONS AND/OR DISPERSIONS
DISPOSITIF D'ÉMULSIFICATION POUR LA PRODUCTION EN CONTINU D'ÉMULSIONS ET/OU DE DISPERSIONS

(30) Priorität: 07.05.2010 DE 102010028774
(43) Veröffentlichungstag der Anmeldung: 13.03.2013
(73) Patentinhaber: OTC GmbH, 46047 Oberhausen (DE)
(72) Erfinder: DAHMS, Gerd, 47138 Duisburg (DE); JUNG, Andreas, 47199 Duisburg (DE); DÖRR, Hendrik, 42489 Wülfrath (DE)
(74) Vertreter: Michalski Hüttermann & Partner Patentanwälte mbB
(86) Internationale Anmeldenummer: PCT/EP2011/057315
(87) Internationale Veröffentlichungsnummer: WO 2011/138438

(56) Entgegenhaltungen:
- EP-A2- 1 964 604
- DE-A1- 1 757 217
- DE-A1- 2 357 212
- DE-A1- 2 720 683
- US-A- 3 807 703
- US-A- 4 155 657
- US-A- 5 149 720

## Beschreibung

Die vorliegende Erfindung betrifft eine Emulgiereinrichtung zur kontinuierlichen Herstellung von Emulsionen und/oder Dispersionen. Die erfindungsgemäße Emulgiereinrichtung kann sowohl für die Herstellung von konventionellen klassischen zweiphasigen Emulsionen, mehrphasigen Emulsionen, wie z. B. multiplen Emulsionen und Dispersionen als auch von dreiphasigen Emulsionen (OW), die neben der dispersen Ölphase noch eine liquid kristalline Gelnetzwerkphase enthalten, aber auch für die Herstellung von flüssig-kristallinen Perlglanzmitteln, flüssig-kristallinen selbstorganisierenden Systemen (Gelnetzwerkphasen in OW-Emulsionen) wie z. B. Haarkonditioniermittel, sowie Haut- und Haar-Reinigungsmitteln wie Shampoos, Duschgele, Wachs- und Silikonemulsionen und Perfluoretheremulsionen etc. eingesetzt werden. Die erfindungsgemäße Emulgiereinrichtung kann in der Putz- und Reinigungsmittelindustrie, der Kosmetikindustrie, Pharmazie, Farben- und Lackindustrie aber auch in der Lebensmittelindustrie eingesetzt werden.

Aus dem Stand der Technik sind Vorrichtungen zur Herstellung von Emulsionen und/oder Dispersionen bekannt, welche in der Regel zur Durchführung von diskontinuierlichen Batch-Verfahren verwendet werden.

WO 2004/082817 A1 offenbart eine Vorrichtung zur kontinuierlichen Herstellung von Emulsionen oder Dispersionen unter Luftausschluss, welche ein allseits geschlossenes Mischvorrichtung umfasst, das Zu- und Abführrohre zum Ein- und Austrag von fließfähigen Stoffen oder Stoffgemischen sowie eine Rührorgan aufweist, das einen Rühreintrag in die Emulsion oder Dispersion ohne Erzeugung von Kavitationskräften und ohne Hochdruckhomogenisierung erlaubt.

EP 1 964 604 A2 offenbart eine Vorrichtung und ein Verfahren zur kontinuierlichen Herstellung einer Mischung aus wenigstens zwei fließfähigen Phasen mit einem allseitig geschlossenen, um seine Längsachse rotationssymmetrischen oder drehsymmetrischen Mischgefäß, wenigstens zwei in das Mischgefäß führenden Zufuhrleitungen zum Eintrag jeweils einer fließfähigen Phase wenigstens einer aus dem Mischgefäß führenden Austragsleitung zum Austragen einer aus diesen Phasen gemischten Mischung und einem rotierbaren Rührer mit Flügelrührern zum Rühren der Phasen, dessen Rotationsachse in der Längsachse des Mischgefäßes liegt. Mit der Vorrichtung nach EP 1 964 604 A2 kann keine kontrollierte Dehnströmung erzeugt werden und es werden keine Maßnahmen zur Verhinderung von Turbulenzen und Kavitationskräften getroffen.

Aufgabe der vorliegenden Erfindung ist es, eine Emulgiereinrichtung, mit deren Hilfe eine kontinuierliche Herstellung von Emulsionen, Nano-Emulsionen und/oder Dispersionen mit flüssigkristalliner Struktur zu schaffen

Erfindungsgemäß wird die Aufgabe gelöst durch eine Emulgiereinrichtung zur kontinuierlichen Herstellung von Emulsionen und/oder Dispersionen gemäß Anspruch 1.

Der perkulierende Mischungsbereich ist der Übergangsbereich der Mischung, in dem diese aus der turbulenten Strömung in die laminare Strömung übergeht. In dem sich an die turbulente Mischung anschließenden perkulierenden Bereich nimmt die Viskosität, hervorgerufen entweder durch ständiges Zerkleinern der Tröpfchen oder durch Ausbilden liquid kristalliner Phasen, zu und die turbulente Strömung ab. Nach Erreichen der kritischen Reynoldszahl geht die Mischung in einen laminaren Mischbereich über. Im laminaren Mischbereich kommt es dann unter Dehnströmungsbedingungen zum kontrollierten und energieeffizienten Tropfenabriss während des Mischvorganges oder der Ausbildung flüssig kristalliner Phasen.

Die Kammer der mindestens einen Mischvorrichtung ist rotationssymmetrisch und weist vorzugsweise die Form eines Hohlzylinders auf. Die Kammer kann aber auch die Form eines Kegelstumpfs, eines Trichters, eines Kuppelstumpfes oder eine aus diesen geometrischen Formen zusammengesetzte Form aufweisen, wobei der Durchmesser der Kammer von der Zulaufleitung zur Ablaufleitung gleich bleibt oder abnimmt. Das Rührorgan ist entsprechend der Form der rotationssymmetrischen Kammer angepasst.

Der Durchmesser der Rührwelle d_{RW} bezogen auf den Innendurchmesser der Kammer d_{K} liegt vorzugsweise im Bereich 0.25 - 0.75 *d_{K} und das Verhältnis zwischen dem Abstand zwischen Zulauf- und Ablaufleitung und der Länge der Arme der Rührelemente liegt vorzugsweise im Bereich 3:1 - 50:1, besonders bevorzugt im Bereich 5:1-10:1, insbesondere im Bereich 6:1 - 8:1. Der ungewöhnlich große Durchmesser der Rührwelle in Bezug auf den Kammerdurchmesser hat weiterhin zur Folge, dass der Abstand zwischen Rührwelle und Kammerwand - vom Fachmann auch als "Fließdurchmesser" bezeichnet - stets so klein ist, dass sich keine trombenartige Strömung ausbilden kann und ein laminarer Fluss gewährleistet ist.

Das Verhältnis von dem Abstand zwischen Zulauf- und Ablaufleitung zum Durchmesser der Kammer am Boden der mindestens einen Mischvorrichtung beträgt ≥ 2:1. Bei einer von einem Hohlzylinder abweichenden Form der rotationssymmetrischen Kammer ist das Verhältnis von Abstand zwischen Zulauf- und Ablaufleitung zum Durchmesser der Kammer im Bereich der Zulaufleitung der mindestens einen Mischvorrichtung ebenfalls ≥ 2:1.

Die Mischvorrichtung ist allseitig geschlossen und wird unter Luftausschluss betrieben. Die zu mischenden Komponenten werden als fließfähige Ströme in die Kammer der Mischvorrichtung eingeleitet, mittels des Rührorgans durchmischt, bis die durchmischten Komponenten die Ablaufleitung erreichen und so abgeleitet werden, dass keine Luft in die Kammer der Mischvorrichtung dringt. Die Mischvorrichtung ist dabei so ausgelegt, dass möglichst wenig Totraum vorhanden ist. Bei der Inbetriebnahme der Mischvorrichtung wird die darin enthaltene Luft durch die eintretenden Komponenten innerhalb von kurzer Zeit komplett verdrängt, wodurch sich das Anlegen eines Vakuums vorteilhaft erübrigt.

Da das System unter Luftausschluss arbeitet und die zu emulgierenden Komponenten kontinuierlich in die Mischvorrichtung eingebracht werden, werden die in der Mischvorrichtung befindlichen Komponenten kontinuierlich in Richtung der Ablaufleitung abtransportiert. Die durchmischten Komponenten durchlaufen die Mischvorrichtung allmählich beginnend von dem Zulauf zu dem Ablauf hin.

In der erfindungsgemäßen Mischvorrichtung durchwandern die über die Zulaufleitungen zugeführten Komponenten nach dem Eintritt in die Kammer zunächst einen turbulenten Mischungsbereich, in dem sie durch die durch die Rührorgane ausgeübten Scherkräfte zunächst turbulent vermischt werden. Hierbei steigt die Viskosität des Mischproduktes bereits merklich an. Weiter in Richtung der Ablaufleitung durchwandert die Mischung dann einen sogenannten perkulierenden Bereich, in dem durch weitere intensive Vermischung die Viskosität der Mischung weiter ansteigt und sich das System allmählich in ein selbstorganisierendes System wandelt. Die Turbulenzen in der in der Mischung vorherrschenden Strömung nehmen mit Erreichen des Perkulationsbereiches allmählich ab, und die Strömungsverhältnisse werden in Richtung der Ablaufleitungen zunehmend laminar. Dadurch entsteht in der Mischung zur Ablaufleitung hin eine lyotrope, flüssigkristalline Phase.

Vorteilhaft ist der Gesamtenergieverbrauch der erfindungsgemäßen Emulgiereinrichtung extrem niedrig. Dieser niedrige Gesamtenergieverbrauch ergibt sich dadurch, dass in den Mischvorrichtungen im Vergleich zu konventionellen Mischverfahren immer nur kleine Volumina gemischt und temperiert werden müssen. Gerade kostenintensive und sehr energieaufwendige Heiz- und Kühlvorgänge sind somit minimiert und tragen entscheidend zum niedrigen Gesamtenergieverbrauch bei. Auch sind die Verweilzeiten des Mischgutes in der Mischkammer sehr kurz. Bei einer Produktionskapazität von 1.000 kg/h beträgt die Verweilzeit im Mittel zwischen 0,5 und 10 Sekunden. Daraus ergibt sich, dass auch die Zuleitungen und Pumpen wesentlich kleiner dimensioniert sind und somit auch die Antriebe der Pumpen wesentlich weniger Energie aufnehmen.

Vorteilhaft trägt das günstige Verhältnis zwischen dem Abstand zwischen Zulauf- und Ablaufleitung und der Länge der Arme der Rührelemente, welches vorzugsweise im Bereich 3:1-50:1, besonders bevorzugt im Bereich 5:1 - 10:1, insbesondere im Bereich 6:1 - 8:1 liegt in Verbindung mit den speziellen Drahtrühren dazu bei, dass eine besonders effektive Drehmomentenausnutzung gewährleistet wird und somit eine gute Durchmischung bei gleichzeitig minimiertem Energieverbrauch des Motors erreicht wird.

Weiterhin ermöglicht der ungewöhnlich große Wellendurchmesser in Bezug auf den Kammerdurchmesser, dass die Rührwelle selbst zur Produkttemperierung genutzt werden kann, was seinerseits zum niedrigen Gesamtenergieverbrauch der erfindungsgemäßen Emulgiereinrichtung beiträgt.

Durch das günstige Verhältnis vom Durchmesser der Kammer zu ihrer Höhe und dem für die Aufrechterhaltung eines laminaren Flusses optimierten Rührorgans ist die Leistungsaufnahme des Rührwerkmotors wesentlich geringer und trägt entscheidend zur niedrigen Gesamtenergieaufnahme der erfindungsgemäßen Vorrichtung bei. Durch die somit im gesamten kleiner dimensionierbaren Bauteile ist eine sehr kompakte und platzsparende Bauweise kennzeichnend für die erfindungsgemäße Mischvorrichtung.

Der Einsatz von Magnetkupplungen trägt ebenfalls zur Verringerung des Gesamtenergieverbrauches bei. Da hier die Kraftübertragung von Motor auf die Motorwelle durch Permanentmagnete erfolgt, muss der Motor nur die Energie aufbringen, welche zur Drehung des Außenrotors benötigt wird. Der Innenrotor mit befestigter Rührwelle wird über die Magnetkraft bewegt. Weiterer Vorteil in Verbindung mit einem Gleitlager ist, dass eine hermetisch abgeschlossene Mischkammer gebaut werden kann.

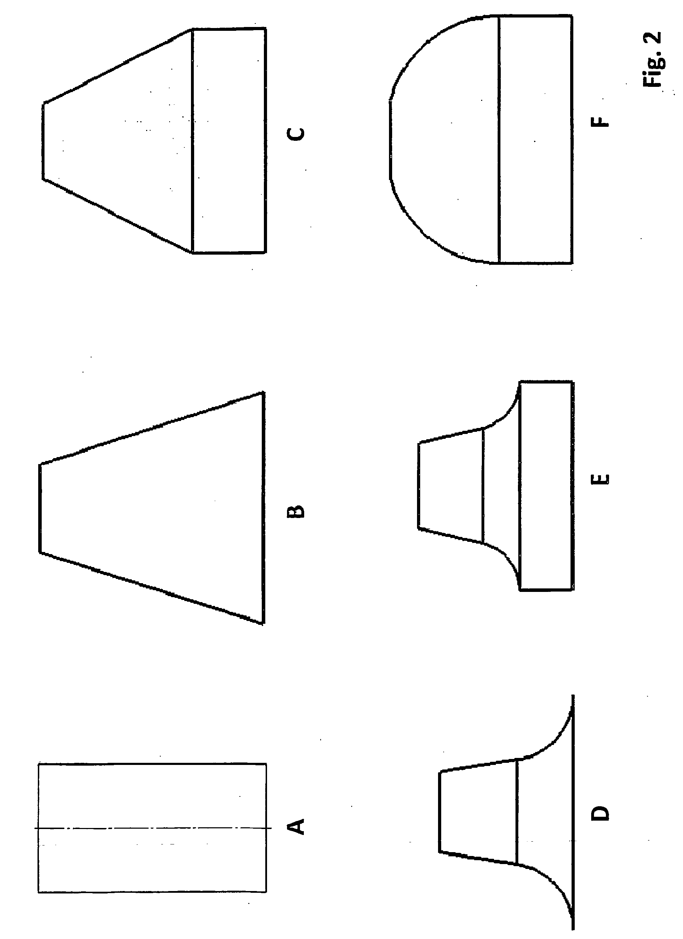

Für ein optimales Emulgierergebnis und zur Vermeidung von Toträumen werden in den erfindungsgemäßen Mischvorrichtungen Kammern, die eine rotationssymmetrische Form aufweisen, eingesetzt. Solche rotationssymmetrischen Formen sind bevorzugt Hohlzylinder **(****Fig. 2 A)****,** aber auch Kegelstumpf **(****Fig. 2 B)****,** Trichter **(****Fig. 2 D)****,** Kuppelstumpf **(****Fig. 2 F)** oder aus diesen zusammengesetzte Formen **(****Fig. 2** **C, E),** bei welchen sich beispielsweise ein kegelstumpfförmiger Bereich an einen hohlzylinderförmigen Bereich anschließt. Der Durchmesser der Mischvorrichtung bleibt dabei vom zulaufseitigen Ende zum ablaufseitigen Ende entweder konstant **(****Fig. 2 A)** oder er nimmt ab **(****Fig. 2** **B - F).**

Besonders bevorzugt wird in der erfindungsgemäßen Mischvorrichtung eine Kammer mit der Form eines Hohlzylinders oder eines Kegelstumpfs oder mit einer zusammengesetzten Form aus einem hohlzylinderförmigen Bereich und einem kegelstumpfförmigen Bereich eingesetzt. Der Kegelstumpf zeichnet sich vorteilhaft dadurch aus, dass der Durchmesser vom zulaufseitigen Ende zum Durchmesser am ablaufseitigen Ende stetig abnimmt, während sich der Durchmesser des Hohlzylinders bezogen auf die Rotationsachse konstant verhält.

Vorteilhaft sind die Kammer der Mischvorrichtung und/oder die Zu- und Ablaufleitungen gemeinsam oder einzeln temperierbar.

Die Komponentenzufuhr in die Mischvorrichtung geschieht durch mindestens eine Zulaufleitung, welche im Durchmesser an die jeweilige Komponente und deren Viskosität angepasst ist und eine vollständige Füllung mit der jeweiligen Phase gewährleistet. Bevorzugt weist die erfindungsgemäße Mischvorrichtung mindestens zwei Zulaufleitungen auf. Im Fall, dass eine Vormischung in die Mischvorrichtung geführt werden soll, kann die Mischvorrichtung aber auch nur eine Zulaufleitung aufweisen. Die zu emulgierenden bzw. zu dispergierenden Komponenten können auch vor dem Eintritt in die Mischvorrichtung beispielsweise über eine Y-förmige Verbindung in eine gemeinsame Zulaufleitung eingeleitet werden, bevor sie die Mischvorrichtung erreichen. In dieser gemeinsamen Zuleitung können sich gegebenenfalls statische Vormischer oder dem Fachmann bekannte passive Mischvorrichtungen befinden. Komponente im Sinne der Erfindung kann ein Reinstoff, aber auch eine Mischung verschiedener Substanzen sein.

Der Eintrittswinkel der Zulaufleitungen in die Mischvorrichtung kann dabei im Bereich von 0° bis 180° bezogen auf die Rotationsachse der Mischvorrichtung liegen. Die Zulaufleitungen können seitlich durch die Mantelfläche oder von unten durch die Bodenfläche in die Kammer reichen.

Die Zulauf- und Ablaufleitungen können auf beliebiger Höhe und auf beliebigem Umfang der Mantelfläche mit der Kammer verbunden sein. Um eine optimale Durchmischung bei gleichzeitig maximaler Verweilzeit der zugeführten Komponenten zu gewährleisten sowie um Toträume zu vermeiden, befindet sich die Eintrittshöhe der Zulaufleitung(en) vorzugsweise im unteren Drittel, bevorzugt im unteren Viertel, der Kammer, bezogen auf die Höhe der Kammer. Die Austrittshöhe der Ablaufleitung befindet sich vorzugsweise im oberen Drittel, bevorzugt im oberen Viertel, der Kammer, bezogen auf die Höhe der Kammer.

Der Durchmesser der Ablaufleitung ist so dimensioniert, dass der Druckaufbau bezogen auf die hohe Viskosität in der mindestens einen oder ersten Mischvorrichtung minimiert wird, jedoch gleichzeitig sichergestellt ist, dass die Ablaufleitungen jederzeit vollständig mit der Mischung gefüllt sind.

Einige Produkte, wie beispielsweise dreiphasige OW-Emulsionen, flüssigkristalline Perlglanzmittel, sowie lyotrope flüssigkristalline Phasen von selbstorganisierenden Systemen, können die zusätzliche zeitversetzte Zugabe von Komponenten in den Perkulationsbereich der ersten Mischvorrichtung verlangen, welcher sich oberhalb der Eintrittshöhe der Zulaufleitungen und unterhalb der Höhe der Austrittsleitungen befindet. Deswegen können sich in diesem Bereich zusätzliche Eintrittsleitungen befinden.

Die Mischvorrichtung kann beliebig orientiert sein, so dass die Drehachse des Rührorgans von horizontal bis vertikal jede gewünschte Position einnehmen kann. Bevorzugt ist jedoch die Mischvorrichtung nicht so angeordnet, dass die Symmetrieachse der Kammer vertikal angeordnet ist und dabei die Zulaufleitungen oberhalb der Ablaufleitungen angebracht sind. Ganz besonders bevorzugt ist die Mischvorrichtung so angeordnet, dass die Symmetrieachse der Kammer vertikal angeordnet ist und dabei die Zulaufleitungen unterhalb der Ablaufleitungen angebracht sind. Der Antriebsmotor treibt hierbei das Rührorgan bevorzugt von oben an, ebenso ist ein Antrieb von unten jedoch möglich.

Überraschenderweise hat sich herausgestellt, dass bei der Geometrie der Mischvorrichtung der Durchmesser der Rührwelle d_{RW} bezogen auf den Innendurchmesser der Kammer d_{K} und das Verhältnis zwischen dem Abstand zwischen Zulauf- und Ablaufleitung und der Länge der Arme der Rührelemente entscheidend ist, um eine optimale Durchmischung der zugeführten Phasen zu gewährleisten. Hierbei hat sich herausgestellt, dass das Verhältnis vom Durchmesser der Rührwelle d_{RW} bezogen auf den Innendurchmesser der Kammer d_{K} vorzugsweise im Bereich 0.25 - 0.75 *d_{K}, besonders bevorzugt im Bereich von 0.3 - 0.7 *d_{K}, insbesondere im Bereich von 0.4 - 0.6 *d_{K} liegt und das Verhältnis zwischen dem Abstand zwischen Zulauf- und Ablaufleitung und der Länge der Arme der Rührelemente vorzugsweise im Bereich 3:1 - 50:1, besonders bevorzugt im Bereich 5:1 - 10:1, insbesondere im Bereich 6:1 - 8:1 liegt.

Dieser ungewöhnlich große Durchmesser der Rührwelle in Bezug auf den Kammerdurchmesser hat weiterhin zur Folge, dass der Abstand zwischen Rührwelle und Kammerwand - vom Fachmann auch als "Fließdurchmesser" bezeichnet - stets so klein ist, dass sich keine trombenartige Strömung ausbilden kann und ein laminarer Fluss gewährleistet ist.

Weiterhin hat sich herausgestellt, dass bei der Geometrie der Mischvorrichtung das Verhältnis zwischen dem Durchmesser der Kammer der Mischvorrichtung und der Strecke, welche die zu mischenden Komponenten vom Zulauf bis zum Ablauf durchwandern müssen, entscheidend ist, um eine optimale Durchmischung der zugeführten Phasen zu gewährleisten. Hierbei hat sich herausgestellt, dass das Verhältnis von Durchmesser zum Abstand zwischen Zulauf und Ablauf vorzugsweise im Bereich von 1:50 bis 1:2, bevorzugt von 1:30 bis 1:3, insbesondere im Bereich von 1:15 bis 1:5 liegt. Durchmesser der Kammer im Sinne der Erfindung ist der Durchmesser am Boden der Kammer.

Das Verhältnis von Durchmesser zum Abstand von Zu- und Ablauf spielt eine entscheidende Rolle zur Kontrolle der Strömung innerhalb der Mischvorrichtung. Denn nur, wenn aus der anfänglich turbulenten Strömung, welche im unteren Bereich der Mischvorrichtung, also im Bereich der Komponentenzuführung, vorliegt, über den sogenannten Perkulationsbereich die Mischung in den laminaren Bereich kommt, ist der Erfolg der Emulgierung gewährleistet. Dabei ist eine exakte Abgrenzung der einzelnen Bereiche nicht möglich, da der Übergang zwischen den jeweiligen Bereichen fließend ist.

Da zur Ausbildung der lyotropen flüssigkristallinen Phase abhängig von den Komponenten unterschiedlich viel Zeit benötigt wird, kann die Mischvorrichtungslänge je nach Produkt angepasst werden. Die Ausbildung der selbstorganisierenden Systeme wird von den folgenden Faktoren beeinflusst: Temperatur innerhalb des Systems, Wassergehalt, Zusammensetzung der Mischung, Strömungsprofil, Scherrate und Verweilzeit.

Die in der erfindungsgemäßen Emulgiereinrichtung und Anlage verwendeten Mischvorrichtungen sind mit Rührorganen ausgestattet, die einen lamellaren Fluss gewährleisten, der die Tröpfchenzerteilung unter laminaren Elongationsbedingungen gewährleistet. Nach einer vorteilhaften Ausgestaltung der Erfindung ist wenigstens ein Bestandteil des Rührelementes beabstandet und parallel zur Innenwandung der Kammer angeordnet.

Bevorzugte Rührorgane sind Vollblatt- oder Teilblattrührer oder Volldraht- oder Teildrahtrührer oder eine Kombination dieser.

Die Tröpfchenzerteilung unter laminaren Elongationsbedingungen führt vorteilhaft zu einer extrem engen Partikelgrößenverteilung um einen mittleren Tröpfchendurchmesser in der erzeugten Emulsion. Sehr oft zeigt der Graph der Partikelgrößenverteilung eine einer Gauß-Kurve sehr ähnliche Form. Die Teilchengrößen, die mit der erfindungsgemäßen Vorrichtung erreichbar sind, liegen je nach Zusammensetzung der Emulsion und/oder Dispersion im Bereich von 50 bis 20.000 nm.

Der Durchmesser des Rührorgans d_{R} bezogen auf den Innendurchmesser der Kammer d_{K} liegt vorzugsweise im Bereich von 0.99 bis 0.6 * d_{K}. Das Rührorgan ist aber mindestens 0.5 mm von der Kammerwand entfernt. Bevorzugt beträgt der Durchmesser des Rührorgans von 0.6 bis 0.7 *d_{K}, besonders bevorzugt von 0.99 bis 0.8 *d_{K}.

Der Durchmesser der Rührwelle d_{RW} bezogen auf den Innendurchmesser der Kammer d_{K} liegt vorzugsweise im Bereich 0.25 - 0.75 *d_{K}, besonders bevorzugt im Bereich von 0.3 - 0.7 *d_{K}, insbesondere im Bereich von 0.4 - 0.6 *d_{K}.

Dieser ungewöhnlich große Durchmesser der Rührwelle in Bezug auf den Kammerdurchmesser hat weiterhin zur Folge, dass der Abstand zwischen Rührwelle und Kammerwand - vom Fachmann auch als "Fließdurchmesser" bezeichnet- stets so klein ist, dass sich keine trombenartige Strömung ausbilden kann und ein laminarer Fluss gewährleistet ist.

Die in der erfindungsgemäßen Vorrichtung einsetzbaren Drahtrührer zeichnen sich dadurch aus, dass auf der Rührwelle Drähte angebracht sind. Es hat sich überraschend herausgestellt, dass mit diesen sehr gute Mischergebnisse und eine minimierte Energieaufnahme erzielt werden, wenn diese in der Art eines Hufeisens bzw. eines Rechtecks mit abgerundeten Ecken gebogen sind und mit ihren Enden mit der Rührwelle verbunden sind.

Auch die Anordnung auf der Welle kann je nach zu vermischendem Produkt unterschiedlich sein. Es können ein oder mehrere hufeisenförmige bzw. rechteckig gebogene Drähte auf der Rührwelle angeordnet werden. Dabei kann entweder ein Volldrahtrührer oder ein Teildrahtrührer eingesetzt werden.

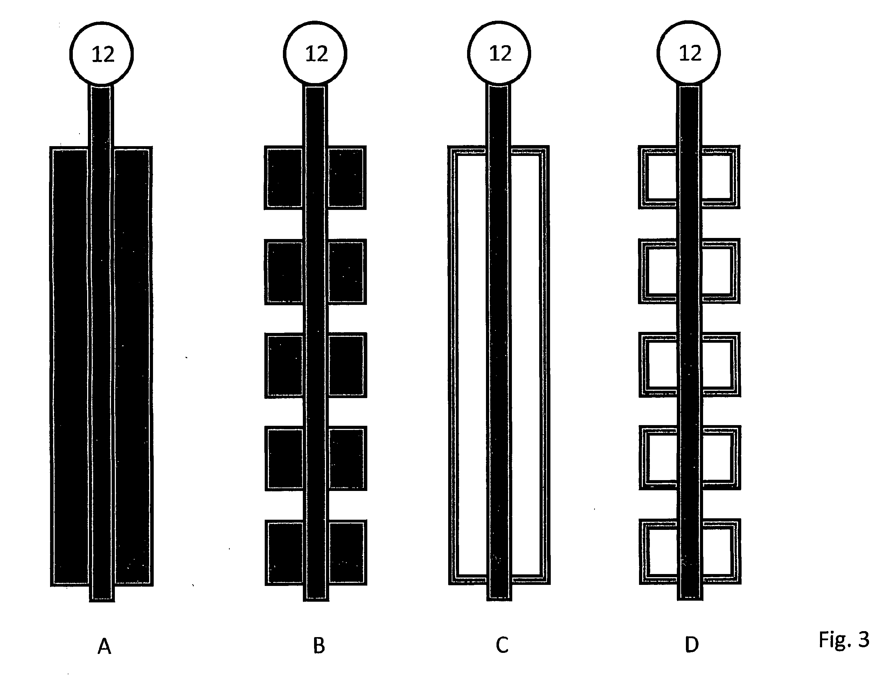

Der Volldrahtrührer **(****Fig. 3 C)** ist dadurch gekennzeichnet, dass er aus mindestens zwei hufeisenförmig bzw. in der Form eines abgerundeten Rechtecks gebogenen Drähten besteht, welche bezogen auf die Welle einander gegenüber an der Welle angebracht sind und im oberen und unteren Bereich der Welle mit dieser verbunden sind. Die Drähte stehen hierbei vorzugsweise senkrecht zur Mittelachse und/oder sind in einem Winkel von 0° bis 90°, bevorzugt von 0° bis 45°, besonders bevorzugt von 0° bis 25°, nach links oder rechts bezogen auf die Rotationsachse gekippt und/oder gedreht. Die obere und untere Länge der Drähte können gleiche oder unterschiedliche Längen aufweisen. Auf dem Umfang der Welle können beliebig viele Drähte angeordnet sein. In dem entstandenen Hohlraum zwischen Welle und Draht können sich weitere Drähte oder beliebige geometrische Formen befinden.

Bevorzugt wird ein Drahtdurchmesser, der maximal im Bereich des Wellendurchmessers liegt und minimal 0,2 mm nicht unterschreitet, besonders bevorzugt ist ein Drahtdurchmesser von maximal 15% des Wellendurchmessers und minimal 0,5mm , insbesondere der Bereich von 10 % des Wellendurchmessers und minimal 1% des Wellendurchmessers.

Der Teildrahtrührer **(****Fig. 3 D)** ist dadurch gekennzeichnet, dass er aus mindestens zwei U- oder hufeisen förmig gebogenen Drähten besteht, deren Enden mit der Welle auf beliebiger Höhe verbunden sind. Die Drähte stehen hierbei vorzugsweise senkrecht zur Mittelachse und/oder sind in einem Winkel von 0° bis 90°, bevorzugt von 0° bis 45°, besonders bevorzugt von 0° bis 25°, nach links oder rechts bezogen auf die Rotationsachse gekippt und/oder gedreht. Die sich radial von der Rührwelle erstreckende obere und untere Länge der Drähte kann gleiche oder unterschiedliche Längen aufweisen. Auf dem Umfang der Welle können beliebig viele Drähte angeordnet sein. In dem entstandenen Hohlraum zwischen Welle und Draht können sich weitere Drähte oder beliebige geometrische Formen befinden.

Bevorzugt wird ein Drahtdurchmesser, der maximal im Bereich des Wellendurchmessers liegt und minimal 0,2 mm nicht unterschreitet, besonders bevorzugt ist ein Drahtdurchmesser von maximal 15 % des Wellendurchmessers und minimal 0,5mm, insbesondere der Bereich von 10 % des Wellendurchmessers und minimal 1% des Wellendurchmessers.

Durch das günstige Verhältnis vom Durchmesser der Kammer zum Durchmesser der Rührwelle in Verbindung mit den vorteilhaften Drahtrührern, wird eine besonders effektive Drehmomentenausnutzung gewährleistet, die die Kraft, welche das Rührorgan auf die zu durchmischenden Komponenten ausübt, minimiert, so dass eine gute Durchmischung bei gleichzeitig minimiertem Energieverbrauch des Motors erreicht wird.

Weiterhin ermöglicht der ungewöhnlich große Wellendurchmesser in Bezug auf den Kammerdurchmesser, dass die Rührwelle selbst zur Produkttemperierung genutzt werden kann.

Desweiteren haben sich Vollblattrührer und Teilblattrührer als besonders geeignet herausgestellt.

Der Vollblattrührer **(****Fig. 3 A)** ist dadurch gekennzeichnet, dass er aus mindestens zwei quadratischen, rechtwinkligen, hufeisenförmigen oder trapezförmigen Blechen besteht, wobei die Ecken der Bleche abgerundet sind, um die Erzeugung von turbulenten Strömungen zur verhindern, wobei eine Seite mit der Welle verbunden ist, und die Bleche durchgehend vom oberen Bereich der Welle bis zum unteren Bereich der Welle reichen. Die Bleche stehen hierbei vorzugsweise senkrecht zur Mittelachse und/oder sind in einem Winkel von 0° bis 90°, bevorzugt von 0° bis 45°, besonders bevorzugt von 0° bis 25°, nach links oder rechts zur Mittelachse gekippt und/oder gedreht. Die oberen und unteren Kanten der Bleche können gleiche oder unterschiedliche Längen aufweisen. Auf dem Umfang der Welle können beliebig viele Bleche angeordnet sein. Die einzelnen Blätter können mit weiteren geometrischen Durchgängen, wie Bohrungen oder Stanzungen versehen sein.

Der Teilblattrührer **(****Fig. 3 B)** ist dadurch gekennzeichnet, dass er aus mindestens zwei quadratischen, rechtwinkligen, hufeisenförmigen oder trapezförmigen Blechen besteht, wobei eine Seite mit der Welle auf beliebiger Höhe verbunden ist. Die Bleche stehen hierbei vorzugsweise senkrecht zur Mittelachse und/oder sind in einem Winkel von 0° bis 90°, bevorzugt von 0° bis 45°, besonders bevorzugt von 0° bis 25°, nach links oder rechts zur Mittelachse gekippt und/oder gedreht. Die oberen und unteren Kanten der Bleche können gleiche oder unterschiedliche Längen aufweisen. Auf dem Umfang der Welle können beliebig viele Bleche angeordnet sein. Die einzelnen Bleche können mit weiteren geometrischen Durchgängen versehen sein.

Weitere dem Fachmann bekannte Rührorgane und ihre Sonderbauformen können zum Vermischen des Produktes im Mischvorrichtung installiert werden, wie z. B. die Bauformen Ankerrührer, Dissolverscheibe, Inter-MIG, etc. Ebenso ist es möglich, verschiedene Rührerbauformen auf einer Rührwelle miteinander zu kombinieren.

Die in der erfindungsgemäßen Mischvorrichtung verwendeten Rührorgane zeichnen sich weiterhin dadurch aus, dass jede Rührwelle rotationsstabil, dazu vorzugsweise im oberen und unteren Bereich der Mischvorrichtung geführt ist. Damit sollen Unwuchten des Rührorgans bei hohen Drehzahlen ausgeschlossen bzw. weitestgehend vermieden werden, so dass keine Turbulenzen erzeugt werden können, die den Aufbau der notwendigen laminaren Strömung beeinflussen oder gar verhindern. Zur Führung der Welle können beispielsweise Kugellager, Linearkugellager, Gleitlager, Lineargleitlager oder dergleichen verwendet werden. Zur weiteren Rotationsstabilität ist die Welle vorteilhaft ausgewuchtet.

Die Materialien, aus welchen sowohl die Mischvorrichtung selbst als auch die oben aufgeführten Rührerbauformen, insbesondere die oben genannten Vollblattrührer, Teilblattrührer, Volldrahtrührer und Teildrahtrührer, gefertigt sind, werden an die chemischen Eigenschaften der zu emulgierenden Komponenten und der entstehenden Emulsionen angepasst. Vorzugsweise umfassen die Rührorgane in der erfindungsgemäßen Mischvorrichtung Stähle, wie beispielsweise rostfreie Edelstähle, aber auch Baustähle, Kunststoffe, wie beispielsweise PEEK, PTFE, PVC oder Plexiglas oder Verbundwerkstoffe oder Kombinationen aus Stahl und Kunststoff.

Die Mischvorrichtungen sind so konzipiert, dass sie von sich aus den zu emulgierenden Komponenten nur einen geringen Gegendruck entgegenstellen. Durch die speziell gebogenen Drahtrührer wird erreicht, dass auch während des Mischvorgangs nur minimaler Druckaufbau entsteht. Aus diesem Grund kann die Mischvorrichtung als im Wesentlichen druckloses/druckarmes System bezeichnet werden.

Um dieses zu erreichen, muss der Querschnitt der Auslaufleitung so gewählt werden, dass die Gesamtproduktmenge der vermischten Komponenten ungehindert abfließen kann. Hierbei ist gerade in der Mischvorrichtung 1 der extreme Viskositätsanstieg zu beachten, welcher beim Aufbau der hochviskosen lyotropen flüssigkristallinen Gelphase entsteht. Auch bei der Dimensionierung weiterer verfahrenstechnischer Bauteile, wie z. B. Rohrleitungen, Wärmetauscher etc., ist darauf zu achten, dass diese dem Gesamtsystem nur minimale Druckabfälle entgegensetzen, um ein durchgehendes druckarmes System zu gewährleisten. Je nach Produkt und Gerätekonfiguration können so Druckabfälle von unter 0,5 Bar im Gesamtsystem realisiert werden.

In der erfindungsgemäßen Emulgiereinrichtung ist eine Temperierung der Mischvorrichtung sowie der Zulauf- und Auslaufleitungen vorteilhaft besonders einfach und effektiv realisierbar. Aufgrund der kleinen Volumina und das durch die Form der Kammer bedingte große Verhältnis von Oberfläche zu Volumen der Kammer in der Mischvorrichtung kann in der erfindungsgemäßen Vorrichtung im Vergleich zu herkömmlichen Emulgiereinrichtungen eine besser kontrollierte Temperaturführung des Produktes garantiert werden.

Zum Heizen der Mischvorrichtungen eignet sich besonders ein Doppelmantel. Dieser kann mit Gasen, wie z. B. Dampf, oder mit Flüssigkeiten, wie z. B. Wasser oder Thermoöl, beheizt werden. Weitere Möglichkeiten sind z. B. elektrische Heizungen wie Heizdrähte, Heizkabel oder Heizpatronen.

Zur Temperierung der zu emulgierenden Komponenten in der Kammer sowie in den Zulauf- und Auslaufleitungen sind sowohl passive Wärmetauschverfahren, wie z. B. Kühlrippen, aktive Verfahren, wie z. B. Rohrbündelwärmetauscher, wie auch Kombinationen beider Methoden einsetzbar, um eine möglichst gleichmäßige und schnelle Temperierung zu gewährleisten.

Zur Temperierung der zu emulgierenden Komponenten von außen nach innen wird die Mischvorrichtung vorzugsweise mit einem Doppelmantel, Voll- oder Halbrohrkühlschlangen ausgerüstet, welche außerhalb und/oder innerhalb der Mischvorrichtung angebracht sind und mit einem Kühl-Heizmedium, z. B. über einen Thermostaten beschickt werden.

Bevorzugt wird die Temperaturführung durch zusätzliche Leitbleche im Inneren des Doppelmantels verbessert. Durch die Optimierung des Verhältnisses von Durchmesser zum Abstand zwischen Zulauf- und Ablaufleitung ist es zusätzlich möglich, den Durchfluss des Mischgutes so anzupassen, dass ein optimaler Temperaturaustausch gegeben ist.

Die erfindungsgemäße Einrichtung zeichnet sich im Gegensatz zu herkömmlichen Batch-Verfahren dadurch aus, dass grundsätzlich nicht alle Komponenten der Rezeptur erwärmt werden müssen, sondern dass nur diejenigen Komponenten, die bei Raumtemperatur nicht ausreichend fließfähig sind, erwärmt werden, bis sie fließfähig sind. Die erfindungsgemäße Ausgestaltung der Mischvorrichtungen, insbesondere das Längen-DurchmesserVerhältnis ist derart vorteilhaft für die Wärmeführung, dass die durch Rühren dissipierte Energie in kontrollierte Wärmezuführung ausgenutzt werden kann.

In einer weiteren Ausführungsform ist die erfindungsgemäße Mischvorrichtung mit Strömungsbrechern ausgestattet, die einen lamellaren Fluss der Komponenten begünstigen.

Nach einer vorteilhaften Ausgestaltung sind die Strömungsbrecher und/oder das Rührorgan temperierbar und ermöglichen damit eine Temperierung der Mischung.

Vorzugsweise umfasst die mindestens eine Mischvorrichtung eine rotationssymmetrische Kammer, in welcher die zu emulgierenden Komponenten über das Durchlaufen eines turbulenten und einer perkulierenden Bereiches in eine lyotrope flüssigkristalline Phase überführt werden.

In einer weiteren Ausführungsform der Erfindung umfasst die mindestens eine Mischvorrichtung mehrere hintereinandergeschaltete, rotationssymmetrische Kammern. Damit wird ermöglicht, dass, wenn aus bautechnischen Gründen die Höhe der mindestens einen Mischvorrichtung begrenzt ist, der Mischvorgang auf mehrere aufeinanderfolgende Kammern aufgeteilt werden kann. Dabei durchlaufen die Komponenten die drei unterschiedlichen Bereiche, Turbulenzbereich, Perkulationsbereich und Laminarbereich nicht innerhalb einer einzigen Kammer, sondern innerhalb mehrerer Kammern.

Die erfindungsgemäße Emulgiereinrichtung umfasst im einfachsten Fall die mindestens eine Mischvorrichtung entsprechend vorgenannter Beschreibung.

Üblicherweise umfasst eine erfindungsgemäße Emulgiereinrichtung jedoch mindestens zwei Mischvorrichtungen, welche in Reihe hintereinander geschaltet sind und in welchen verschiedene Komponenten nacheinander oder gleichzeitig zugeführt und miteinander vermischt werden. Dabei ist die Viskosität der in der ersten Mischvorrichtung erzeugten Mischung immer größer oder gleich der Viskosität in der (den) nachfolgenden Mischvorrichtung(en). Zumindest die erste Mischvorrichtung muss dabei in Aufbau und Funktion der mindestens einen Mischvorrichtung entsprechen, d. h. in der ersten Mischvorrichtung muss die besondere Strömungsführung gewährleistet sein, bei der die Komponenten zunächst turbulent vermischt werden und dann über das Durchlaufen eines perkulierenden Bereiches einen lyotropen flüssigkristallinen Zustand erreichen.

Bei der Herstellung klassischer zweiphasiger Systeme wie WO-Emulsionen, aber auch OW-Emulsionen ohne Gelnetzwerkphase, in der erfindungsgemäßen Emulgiereinrichtung ist das Verhältnis von interner (disperser) Phase und externer (kontinuierlicher) Phase in der ersten Mischvorrichtung immer größer als in der (den) nachfolgenden Mischvorrichtung(en).

In der erfindungsgemäßen Emulgiereinrichtung ist es desweiteren möglich, dass mehrere Mischvorrichtungen nicht nur in Reihe hintereinandergeschaltet werden, sondern auch seriell über- oder untereinander geschaltet werden können. Dabei können auch die einzelnen Mischvorrichtungen zusammen in einem Gehäuse untergebracht sein, so dass von außen die Trennung der Mischvorrichtungen nicht sichtbar ist.

Im weiteren Verlauf der Herstellung der genannten Produkte in der erfindungsgemäßen Emulgiereinrichtung wird der hochviskose Inhalt aus der ersten Mischvorrichtung in die nachfolgende(n) Mischvorrichtung(en) geleitet. Dabei wird die Zuführung in die nachfolgenden Mischvorrichtungen so gestaltet, dass die Höhe der Eintrittsleitungen vorzugsweise im unteren Drittel, bevorzugt im unteren Viertel, bezogen auf die Höhe der Mischvorrichtung erfolgt.

In den der ersten Mischvorrichtung nachgeschalteten Mischvorrichtungen ist es nicht mehr notwendig, dass die interne Phase im Verhältnis zur kontinuierlichen Phase überwiegt. In einer Ausführungsform der erfindungsgemäßen Emulgiereinrichtung werden in einer ersten Mischvorrichtung die zu emulgierenden Komponenten in eine laminar flüssigkristalline Phase überführt und in einer zweiten Mischvorrichtung durch die Zugabe von externer Phase auf die gewünschte Konzentration verdünnt. Die erfindungsgemäße Emulgiereinrichtung umfasst auch entsprechende Peripherie, wie
Vorratsbehälter für mindestens 2 Komponenten
Verbindungsleitungen für die Zuführung der Komponenten zu der mindestens einen Mischvorrichtung, zugehörige Pumpen und Ventile,
Verbindungsleitungen für die Abführung von Komponenten,
Steuerungseinrichtung zur Überwachung und Regelung der Prozessstufen,
eine Anzeigeeinrichtung mit einem Bedienteil zur Visualisierung und Eingabe von Prozessvariablen.

Mischvorrichtungen und Verbindungsleitungen sind temperierbar.

Mischvorrichtung und Verbindungsleitungen können Sensoren zur Produkt- und Prozesskontrolle aufweisen.
Weiterhin können die Auslaufleitungen der einzelnen Mischvorrichtungen weitere Sensoren aufweisen, die z. B. eine kontinuierliche Partikelgrößenmessung, direkt oder im Bypass, eine Temperaturmessung, eine Druckmessung, eine Leitfähigkeitsmessung, eine Viskositätsmessung, oder ähnliches ermöglichen.

Die Produktqualität des Endprodukts wird in der erfindungsgemäßen Einrichtung vorrangig in der ersten Rührstufe bestimmt.

Weiterhin kann in die Zulauf- und Auslaufleitung der erfindungsgemäßen Mischvorrichtungen oder bei mehreren Mischvorrichtungen, zwischen den Mischvorrichtungen einer erfindungsgemäßen Anlage, ein Wärmetauscher angebracht werden. Es hat sich gezeigt, dass hier das Einbringen von Rohrbündelwärmetauschern in Verbindung mit verdrillten Leitblechen im Produktstrom und Leitblechen im Heiz- und Kühlkreislauf sehr effektiv ist. Durch die verhältnismäßig geringen Produktmengen ist vorteilhaft eine sehr kompakte und effiziente Bauweise der Wärmetauscher möglich. Diese Wärmetauscher sind sowohl bei der seriellen Bauweise als auch bei der in Reihe geschalteten Bauweise einsetzbar. Das Einbringen von anderen Wärmetauscher-Bauformen, wie z. B. Kühlschlangen, Rohrbündelwärmetauscher, Doppelrohr-Wärmetauscher, Rippenrohr-Wärmetauscher, Spiralbandwärmetauscher, Plattenwärmetauscher, Speicherwärmetauscher und weitere Sonderbauformen, ist ebenfalls möglich.

Als Kühlmedium sind sowohl Gase, wie z. B. Stickstoff, als auch Flüssigkeiten, wie z. B. Wasser oder Thermoöl, einsetzbar.

Mit den oben genannten Wärmetauschern kann ebenso gekühlt wie auch geheizt werden. Auch hier kann vom Fachmann eine geeignete Beheizung/Kühlung für das gewünschte Produkt gewählt werden.

Je nach Einsatz der Emulgiereinrichtung ist gegebenenfalls auch eine Kombination aus Heiz- und Kühleinheiten möglich. Auch dies kann durch Einsatz eines Doppelmantels, einer Heiz-/Kühlschlange oder eines entsprechenden Wärmetauschers wie oben beschrieben einfach und effizient gelöst werden.

In kleineren Emulgiereinrichtungen eignen sich hierzu besonders Heiz-/Kühlbäder (Thermostate), welche vorzugsweise durch eine übergeordnete Steuerung überwacht und betrieben werden. Zusätzlich kann mit diesen Thermostaten auch ein Stand-Alone Betrieb ermöglicht werden. Da die Thermostate in der Regel auch die Möglichkeit besitzen, einen externen Temperaturfühler anzuschließen, kann dieser in den Produktstrom eingebracht werden. Das Thermostat regelt dann die benötigte Heiz- bzw. Kühlleistung selbstständig und sorgt so für eine optimale Produkttemperatur. Weiterer Vorteil dieser Methode ist eine Entlastung der Steuerung, da diese die Regelung der Temperatur der Mischvorrichtungen an das Thermostat abgeben kann.

Durch eine Optimierung der Temperatur der Komponentenzuführung in die Mischvorrichtungen ist ebenfalls eine Optimierung der Produkttemperatur erreichbar. Hierbei kann auch der Zuflussweg der Komponenten vom Vorratsbehälter bis zum Eintritt in die Mischvorrichtung soweit optimiert und genutzt werden, dass Komponentenströme mit einerfür die zu emulgierenden Komponenten optimalen Temperatur in die Mischvorrichtung gelangen.

Eine erfindungsgemäße Emulgiereinrichtung umfasst
- mindestens eine erfindungsgemäße Mischvorrichtung
- mindestens einen Motor für die Rührorgane der Mischvorrichtung,
- mindestens zwei Vorratsgefäße für die zu emulgierenden Phasen, die mit der Mischvorrichtung über die Zulaufleitungen verbunden sind, und aus welchen mittels Fördereinrichtungen die Komponenten luftfrei in die Mischvorrichtung geführt werden,
- mindestens eine Fördereinrichtung je Komponente oder je Komponentenmischung,
- ggf. Eingangsstromüberwachungssensoren und/oder Ausgangsstrom-Überwachungssensoren, mit welchen ggf. gleichzeitig eine automatische Qualitätskontrolle durchgeführt werden kann,
- ggf. mindestens eine Einrichtung zur Temperierung für die Emulgiereinrichtung und das Leitungssystem für Zu- und Ableitung der Komponenten, Komponentenmischungen,
- eine Steuerungseinrichtung für die Überwachung und Steuerung der Mischvorrichtungen, die Zu- und Ableitung der Komponenten, Komponentenmischungen,
- ggfls. eine Anzeigeeinrichtung mit einem Bedienfeld zur Visualisierung und zur Eingabe von Daten.

Üblicherweise umfasst die Emulgiereinrichtung jedoch mindestens zwei Mischvorrichtungen, welche hintereinander geschaltet sind und in welchen aufeinanderfolgend verschiedene Komponenten miteinander vermischt werden. Dabei ist die Viskosität der in der ersten Mischvorrichtung erzeugten Mischung immer größer oder gleich der Viskosität in der (den) nachfolgenden Mischvorrichtung(en). Zumindest die erste Mischvorrichtung muss dabei in Aufbau und Funktion der mindestens einen Mischvorrichtung entsprechen, d. h. in der ersten Mischvorrichtung muss die besondere Strömungsführung gewährleistet sein, bei der die Komponenten zunächst turbulent vermischt werden und dann über das Durchlaufen eines perkulierenden Bereiches einen lyotropen flüssigkristallinen Zustand erreichen.

Bei der Herstellung klassischer zweiphasiger Systeme wie WO-Emulsionen, aber auch OW-Emulsionen ohne Gelnetzwerkphase, in der erfindungsgemäßen Emulgiereinrichtung ist das Verhältnis von interner (disperser) Phase und externer (kontinuierlicher) Phase in der ersten Mischvorrichtung immer größer als in der (den) nachfolgenden Mischvorrichtung(en).

Die gesamte erfindungsgemäße Anlage wird über eine speicherprogrammierbare Steuerung gesteuert. Diese überwacht z. B. die Drehzahlen der Mischvorrichtungen, den Zufluss der Einzelkomponenten, die Drehzahlen der Pumpen, die Temperaturen und Drücke der einzelnen zugefügten Phasen und alle weiteren für den Betrieb erforderlichen Parameter. Sie kann in Verbindung mit Masse- oder Volumendurchflussmessern den Zufluss der einzelnen Komponenten in die jeweiligen Mischvorrichtungen überwachen und regeln. Sie kann vorher definierte Warnungen und Störungen über ein optisches oder akustisches Ausgabegerät weitergeben. Optische und visuelle Ausgabe können sich dabei getrennt von der erfindungsgemäßen Vorrichtung befinden wie z. B. in einem Leitstand.

Alternative Steuerungsmöglichkeiten, wie z. B. Software SPS oder PC-Steuerung sind ebenso möglich wie eine Kombination aus mehreren Steuerungsmöglichkeiten.

Über ein mit der Steuerungseinrichtung integriertes oder diesem angeschlossenes Fernwartungsmodul zum Anschlusses einer analogen Telefonleitung oder einer ISDN-Leitung, den Zugang zu einem Mobilfunknetz oder eines LAN oder WLAN Netzes ist es möglich, eine Fernwartung der erfindungsgemäßen Vorrichtung vorzunehmen oder auch Warn- und Störmeldungen zu versenden oder die gesamte erfindungsgemäße Anlage zu steuern.

Weiterhin kann die Steuerung ein Rezepturmodul aufweisen, bei dem eine oder mehrere Rezepturen für diverse Produkte hinterlegt sind. Jede Rezeptur kann hierbei aus mehreren Datensätzen bestehen. In den Datensätzen werden die für den Betrieb notwendigen Parameter wie z. B. die Drehzahl, das Verhältnis der Volumenströme etc. festgehalten. Nach Aufruf der Rezeptur werden die Datensätze entweder zeitgesteuert, oder nach Auslösen eines bestimmten Ereignisses, z. B. das Erreichen einer bestimmten Temperatur, abgearbeitet. Dies ermöglicht die Sicherstellung, dass Produkte mit immer der gleichen Qualität produziert werden können.

Anhand nachfolgender Figuren und Ausführungsbeispiele wird die Erfindung näher erläutert, ohne diese einzuschränken. Dabei zeigen
- Fig. 1: Emulgiereinrichtung mit einer Mischvorrichtung
- Fig. 2: Verschiedene Mischvorrichtungsgeometrien
- Fig. 3: Verschiedene Rührorgane
- Fig. 4: Emulgiereinrichtung mit einer Mischvorrichtung mit einer weiteren Zuführungsleitung im Perkulationsbereich
- Fig. 5: Emulgiereinrichtung mit zwei Mischvorrichtungen
- Fig. 6: Emulgiereinrichtung mit zwei Mischvorrichtungen und Wärmetauscher
- Fig. 7: Anlagenschema
- Fig. 8: Energiediagramm

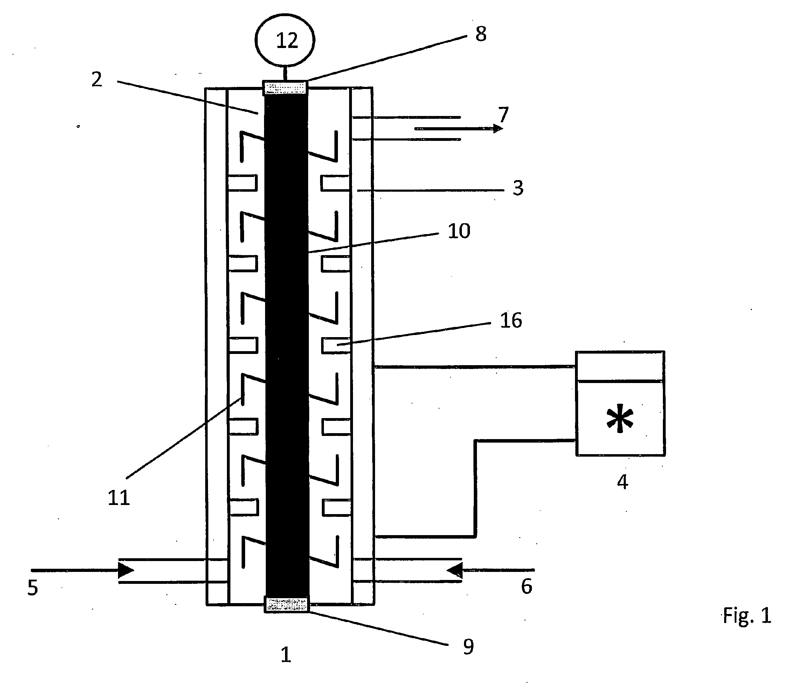

**Fig. 1** zeigt in Schnittdarstellung eine Emulgiereinrichtung mit einer Mischvorrichtung 1 mit einer allseitig geschlossenen rotationssymmetrischen Kammer 2 in Form eines Hohlzylinders. In die Kammer ragt eine Rührwelle 10, auf der Rührdrähte 11, wie in Fig. 3D dargestellt angeordnet sind. Die Rührwelle 10 wird vom Motor 12 getrieben und von den Lagern und Dichtungen 8 geführt. Weiterhin ist die Rührwelle 10 im Bodenteil der Kammer 2 zusätzlich im Lager 9 geführt. Die Kammer 2 weist im unteren Teil Zulaufleitungen 5 bzw. 6 für den luftfreien Zulauf der zu emulgierenden Komponenten A und B auf. Im oberen Teil der Kammer 2 ist die Ablaufleitung 7 angeordnet. Zu- und Ablaufleitungen sind ebenfalls temperiert und weisen entsprechende Förderpumpen auf (in Fig. 1 nicht dargestellt).

Das Verhältnis zwischen dem Abstand zwischen Zulaufleitungen 5 bzw. 6 und Ablaufleitung 7 und dem Durchmesser der Kammer 2 beträgt etwa 3,5.

Das Verhältnis zwischen dem Abstand zwischen Zulaufleitungen 5 bzw. 6 und Ablaufleitung 7 und der Länge der Rührarme der Drahtrührer beträgt etwa 15:1.

Die Kammer 2 ist von einem Thermostatmantel 3 umgeben, der in Verbindung mit dem Thermostat 4 eine Temperierung des Mischgutes erlaubt. Aufgrund des gegenüber dem Kammerdurchmesser größeren Abstandes zwischen Zu- und Ablauf kann das Mischgut kontrolliert so temperiert werden, dass der durch den Rührer verursachte Energieeintrag das Mischgut nicht destabilisiert.

Die Emulgiereinrichtung nach Fig. 1 kann z. B. für die Verdünnung von 100 Kg pro Stunde Natrium Lauryl Ethersulfat (SLES) wie folgt genutzt werden:
Über die Pumpe der Phase A werden über die Zulaufleitung 5 kontinuierlich 41.4 kg pro Stunde 70%iges SLES und über die Zulaufleitung 6 mittels Pumpe der Phase B kontinuierlich 58.6 kg pro Stunde Wasser in die Mischvorrichtung 1 geleitet und mit 3000 Umdrehungen pro min vermischt.

Die Mischvorrichtung 1 ist allseitig geschlossen und wird unter Luftausschluss betrieben. Die zu mischenden Komponenten A und B werden als fließfähige Ströme in die Kammer 2 der Mischvorrichtung 1 eingeleitet, mittels des Rührorgans 10 mit den Rührdrähten 11 durchmischt bis die durchmischten Komponenten die Ablaufleitung 7 erreichen und so abgeleitet werden, dass keine Luft in die Kammer 2 der Mischvorrichtung 1 dringt.

Bei der Inbetriebnahme der Mischvorrichtung wird die darin enthaltene Luft durch die eintretenden Komponenten A und B innerhalb kurzer Zeit komplett verdrängt, wodurch sich das Anlegen eines Vakuums vorteilhaft erübrigt.

Die durchmischten Komponenten A und B durchlaufen die Kammer 2 der Mischvorrichtung 1 allmählich beginnend vom Zulauf 5, 6 zum Ablauf 7 hin. Die über die Zulaufleitungen 5, 6 in die Kammer 2 eingebrachten Komponenten A und B durchwandern zunächst einen zulaufseitigen turbulenten Mischungsbereich, in dem sie durch die durch die Rührdrähte 11 ausgeübten Scherkräfte turbulent vermischt werden. In einem sich darüber anschließenden perkulierenden Mischungsbereich werden die Komponenten weiter durchmischt, wobei die turbulente Strömung ab- und die Viskosität zunimmt, bis sich in einem ablaufseitigen laminaren Mischungsbereich eine lyotrope, lamellar-flüssigkristalline Phase einstellt. Über den Thermostatmantel 3 wird die Temperatur der Mischung konstant gehalten.

Am Ausgang der Rührstufe erhält man 28%iges SLES.

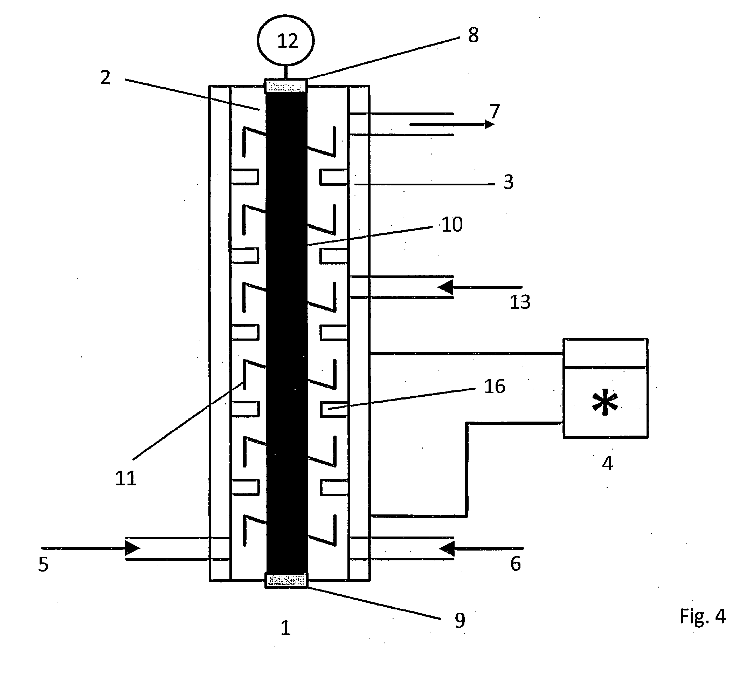

**Fig. 4** zeigt in Schnittdarstellung eine einstufige Emulgiereinrichtung, die analog Fig. 1 aufgebaut und dimensioniert ist, aber eine weitere Zulaufleitung 13 für eine Komponente C aufweist. Zu- und Ablaufleitungen sind temperiert und stehen mit Pumpen in Wirkverbindung (in Fig. 4 nicht dargestellt).

Die Emulgiereinrichtung nach Fig. 4 kann für die Herstellung eines einfachen O/W-Sprays wie folgt genutzt werden.

Komponente A: Wässrige Emulgatorphase
Komponente B: Ölphase
Komponente C: Wasserphase

Komponente A wird mit 8.1 kg pro Stunde kontinuierlich über die Zulaufleitung 5 und Komponente B mit 22.5 kg pro Stunde über die Zulaufleitung 6 in Kammer 2 der Mischvorrichtung 1 luftfrei eingeleitet und mit etwa 3000 Umdrehungen pro min vermischt. Mittels des Rührorgans 10 mit den Rührdrähten 11 werden die Komponenten A und B durchmischt. Nachdem die Mischung etwa 60 % der Kammerlänge durchlaufen hat, wird die Komponente C über die Zulaufleitung 13 in die Mischkammer mit 69.4 kg pro Std. zudosiert und vermischt, bis die durchmischten Komponenten die Ablaufleitung 7 erreichen. Bei der Inbetriebnahme der Mischvorrichtung 1 wird die darin enthaltene Luft durch die eintretenden Komponenten innerhalb kurzer Zeit komplett verdrängt, wodurch sich das Anlegen eines Vakuums vorteilhaft erübrigt.

Die durchmischten Komponenten A und B durchlaufen die Mischvorrichtung 1 allmählich beginnend vom Zulauf 5, 6 zum Ablauf 7 hin. Die über die Zulaufleitungen 5, 6 in die Kammer 2 eingebrachten Komponenten A und B durchwandern zunächst einen zulaufseitigen turbulenten Mischungsbereich, in dem sie durch die durch die Rührdrähte 11 ausgeübten Scherkräfte turbulent vermischt werden. In einem sich darüber anschließenden perkulierenden Mischungsbereich werden die Komponenten A und B weiter durchmischt, wobei die turbulente Strömung ab- und die Viskosität zunimmt, bis sich in einem ablaufseitigen laminaren Mischungsbereich eine lyotrope, flüssigkristalline Phase einstellt und in welchen die Komponente C über die Zulaufleitung 13 zugeführt wird. Über den Thermostatmantel 3 wird die Temperatur der Mischung konstant gehalten.

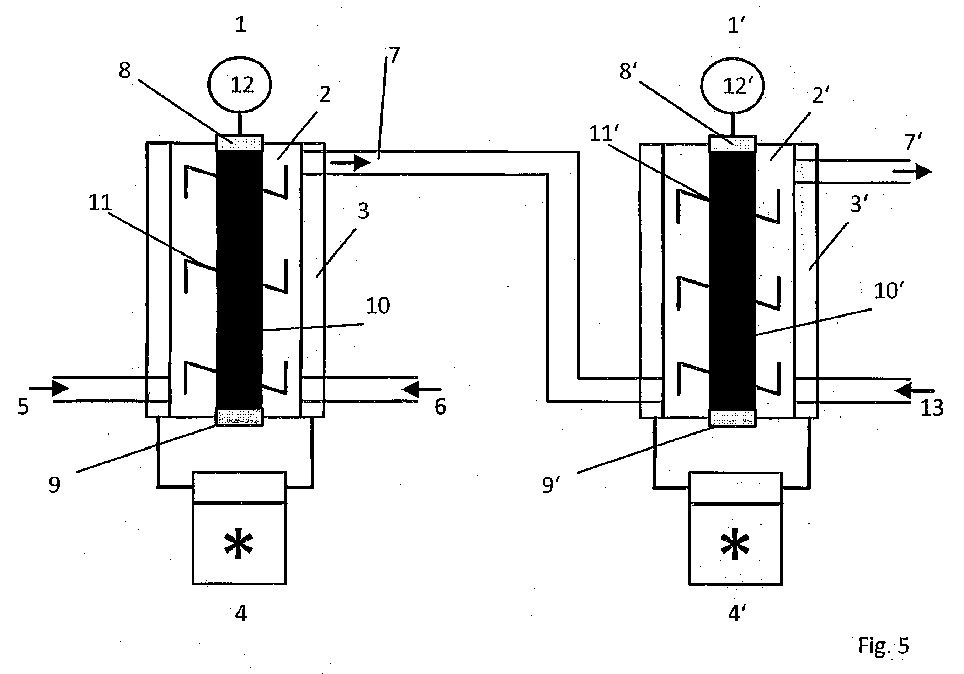

**Fig. 5** zeigt in Schnittdarstellung eine Emulgiereinrichtung mit zwei Mischvorrichtungen 1 und 1'.

Die Emulgiereinrichtung nach Fig. 5 zeichnet sich dadurch aus, dass sie aus zwei in Reihe geschalteten Mischvorrichtungen 1 und 1' besteht, wobei die Auslaufleitung 7 der ersten Mischvorrichtung 1 mit der Zulaufleitung der nachfolgenden Mischvorrichtung 1' verbunden ist. Jede Mischvorrichtung 1 und 1' besitzt einen Thermostatmantel 3 bzw. 3' und kann, wenn gewünscht, über die Thermostaten 4 bzw. 4' einzeln temperiert werden. Rührelemente sind auf der Rührwelle befestigte Drahtrührer entsprechend Darstellung Fig. 3D.

Das Verhältnis zwischen dem Abstand zwischen Zulaufleitungen 5 bzw. 6 und Ablaufleitung 7 und dem Durchmesser der Kammer 2 der Mischvorrichtung 1 beträgt etwa 2,0.

Das Verhältnis zwischen dem Abstand zwischen Zulaufleitungen 5 bzw. 6 und Ablaufleitung 7 und der Länge der Rührarme der Drahtrührer beträgt 8:1.

Kammer 2' der Mischvorrichtung 1' entspricht im Aufbau und Dimensionierung der Kammer 2 der Mischvorrichtung 1.

Die Mischvorrichtungen 1 bzw. 1' sind mit Sensoren für Viskosität, Druck, Temperatur (hier nicht dargestellt) ausgestattet. Die Mischvorrichtungen 1 und 1' sind allseitig geschlossen.

Die Emulgiereinrichtung nach Fig. 5 kann für die Herstellung einer einfachen OW-Emulsion (120 kg pro Stunde) wie folgt genutzt werden.
Komponente A: Emulgator mit zusätzlicher Base zur Neutralisation des Verdickers
Komponente B: Ölphase
Komponente C: Wasserphase mit Verdicker

Komponente A wird mit 5.65 kg pro Stunde kontinuierlich über die Zulaufleitung 5 und Komponente B mit 21.93 kg pro Stunde über die Zulaufleitung 6 in die Kammer 2 der Mischvorrichtung 1 eingeleitet und mit etwa 3000 Umdrehungen pro min vermischt. Mittels des Rührorgans 10 mit den Rührdrähten 11 werden die Komponenten A und B durchmischt bis die durchmischten Komponenten die Ablaufleitung 7 erreichen und so in die Kammer 2' der Mischvorrichtung 1' abgeleitet werden, dass keine Luft in die Kammer 2 der Mischvorrichtung 1 dringt. Bei der Inbetriebnahme der Mischvorrichtung 1 und 1' wird die darin enthaltene Luft durch die eintretenden Komponenten innerhalb kurzer Zeit komplett verdrängt, wodurch sich das Anlegen eines Vakuums vorteilhaft erübrigt.

Die durchmischten Komponenten A und B durchlaufen die Mischvorrichtung 1 allmählich beginnend vom Zulauf 5, 6 zum Ablauf 7 hin. Die über die Zulaufleitungen 5, 6 in die Kammer 2 eingebrachten Komponenten A und B durchwandern zunächst einen zulaufseitigen turbulenten Mischungsbereich, in dem sie durch die durch die Rührdrähte 11 ausgeübten Scherkräfte turbulent vermischt werden. In einem sich darüber anschließenden perkulierenden Mischungsbereich werden die Komponenten A und B weiter durchmischt, wobei die turbulente Strömung ab- und die Viskosität zunimmt, bis sich in einem ablaufseitigen laminaren Mischungsbereich eine lyotrope, lamellar-flüssigkristalline Phase einstellt. Über den Thermostatmantel 3 wird die Temperatur der Mischung konstant gehalten.

Phase C wird über die Zulaufleitung 13 in die Kammer 2' mit 72.42 kg pro Stunde gemeinsam mit der hochviskosen Mischung der Komponenten A und B eingeleitet. Mittels Rührorgan 10 und Rührdrähten 11 werden die Komponenten durchmischt bis sie die Ablaufleitung 7' erreichen und so abgeführt werden , das keine Luft in die Kammer 2' dringt.

In der Kammer 2' wird die hochviskose Mischung der Komponenten A und B mit der Wasserphase der Komponente C zu einer fließfähigen Emulsion mit einer Partikelgröße von 400 nm und einer Viskosität von 15.000 m Pas verdünnt. Der Verdicker dient dabei zur Emulsionsstabilisierung und beeinflusst das Hautgefühl positiv.

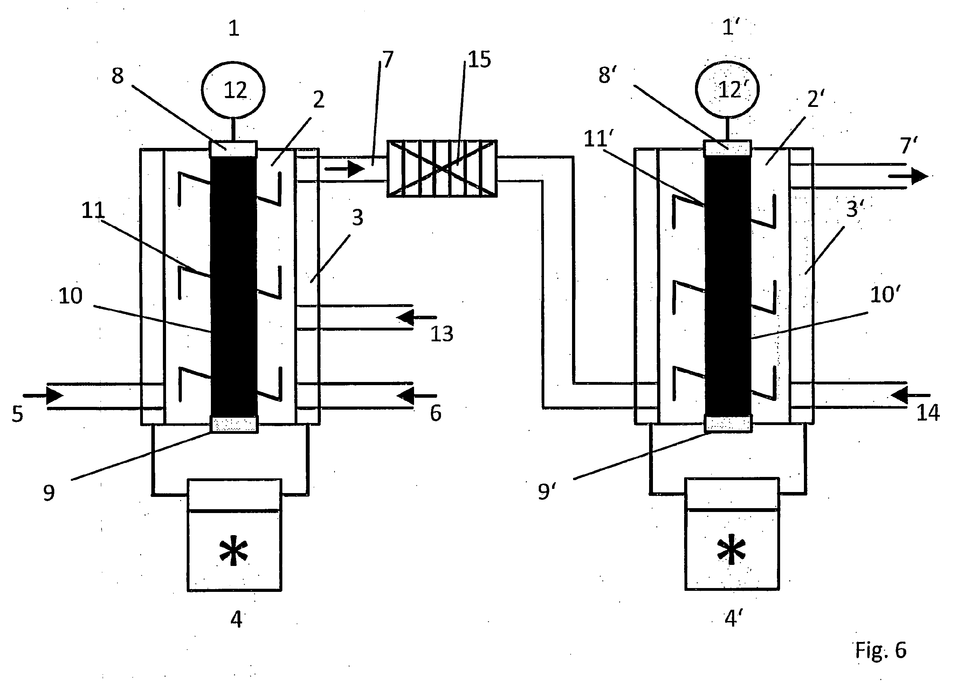

Fig. 6 zeigt in Schnittdarstellung eine Emulgiereinrichtung mit zwei Mischvorrichtungen 1 und 1' und einem zwischengeschalteten Plattenwärmetauscher 15. Die Emulgiereinrichtung nach Fig. 6 ist analog der Emulgiereinrichtung nach Fig. 5 aufgebaut und dimensioniert. Unterschiedlich ist die zusätzliche Zulaufleitung 13 für die Komponente C sowie der Plattenwärmetauscher 15 in der Ablaufleitung 7 zum Zulauf in die Kammer 2.

Die Mischvorrichtungen 1 und 1' sind jeweils mit einem heiz-/kühlbarem Thermostatmantel 3 und 3' ausgestattet und in Reihe geschaltet. Zwischen den beiden Mischvorrichtungen 1 und 1' kann das Produkt durch einen Plattenwärmetauscher zusätzlich beheizt und gekühlt werden.

Die Emulgiereinrichtung nach Fig. 6 kann für die Herstellung eines Perlglanzmittels (100 kg pro Stunde) wie folgt genutzt werden.

| Komponente | Komponente | Kesseltemperatur | Durchsatz |
|---|---|---|---|
| A | SLES | Raumtemperatur (RT) | 22 kg pro Stunde |
| B | Glycol Distearate | 70°C | 24 kg pro Stunde |
| C | Wasser, Betain (Co-Tensid) | RT | 21 kg pro Stunde |
| D | Wasser und Konservierungsmittel | RT | 33 kg pro Stunde |

| | |
|---|---|
| Temperatur Strang Phase A: | RT |
| Temperatur Strang Phase B: | 80°C |
| Temperatur Strang Phase C: | RT |
| Temperatur Strang Phase D: | RT |

| | |
|---|---|
| Temperatur Rührstufe 1 | 65°C |
| Temperatur Rührstufe 2: | 5°C |
| Temperatur Wärmetauscher: | 40°C |

| | |
|---|---|
| Rührstufe 1: | 3000 U/min. |
| Rührstufe 2: | 3000 U/min. |

Komponente A wird mit 22 kg pro Stunde und mit Raumtemperatur kontinuierlich über die Zulaufleitung 5 und Komponente B mit 24 kg pro Stunde mit einer Temperatur von 80 °C über die Zulaufleitung 6 in die Kammer 2 der Mischvorrichtung 1 eingeleitet und mit etwa 3000 Umdrehungen pro min vermischt. Die Zulaufleitung 6 ist temperiert, so dass Komponente B erwärmt und mit einer Temperatur von 80 °C in die Kammer 2 geleitet wird.

Wenn die mittels des Rührorgans 10 mit den Rührdrähten 11 durchmischten Komponenten A und B den Bereich der Zulaufleitung 13 erreichen, wird über die Zulaufleitung 13 die Komponente C mit 21 kg pro Stunde und einer Temperatur von 65 °C der Mischung zugeleitet. Der Thermostatmantel 3 der Kammer 2' ist über den Thermostaten 4' auf 65 °C temperiert, so dass die Komponenten A, B und C bei 65 °C durchmischt werden.

Nach Zuleitung von Komponente C geht die Mischung in einen perkulierenden Bereich über, bis sie im Bereich der Ablaufleitung 7 einen lyotrope, flüssigkristalline Zustand erreicht.

Bevor die über Ablaufleitung 7 abgeführte lyotrope, flüssigkristalline Mischung der Kammer 2' zugeführt wird, wird diese Mischung mittels des in die Leitung 7' geschalteten Plattenwärmetauschers 15 auf 40 °C abgekühlt. Dies ist notwendig, da die liquid-kristalline Vorstufe, die in der Mischvorrichtung 1 hergestellt wird, temperaturempfindlich ist. Danach wird die liquid-kristalline Vorstufe in der zweiten Mischvorrichtung 1' unter Gegenkühlung durch den Heiz/Kühlmantel bei einer Temperatur von 5 °C mit der Phase D verdünnt. Die Produktqualität kann nur durch Einhaltung dieses Temperaturprofils erreicht werden. Würde man oberhalb von 40 °C mit der kalten Phase D verdünnen, so könnten die Qualitätsanforderungen an das Produkt nicht erfüllt werden. Kühlt man das Produkt vor dem Verdünnen zu tief ab, erhält man ebenfalls ein Produkt, das nicht den Qualitätsansprüchen genügt. Dies ist der Tatsache geschuldet, dass die liquid-kristalline Vorstufe in Abhängigkeit von der Temperatur unterschiedliche liquid-kristalline Strukturen annimmt, aus welchen bei Verdünnung unterschiedliche Endzustände erreicht werden.

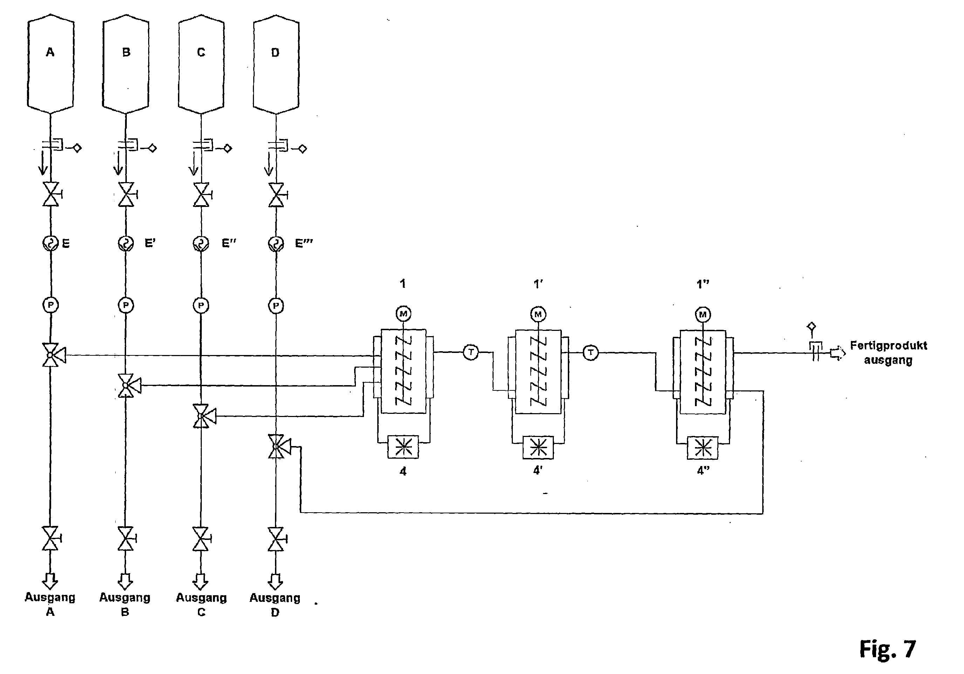

In **Fig. 7** ist ein Schema einer kompletten Emulgieranlage zur Herstellung eines Shampoos dargestellt. Die Emulgieranlage umfasst 3 Mischvorrichtungen 1, 1' und 1", Vorratsbehälter A bis D für die zu mischenden Komponenten A bis D, Verbindungsleitungen für die Zuführung der Komponenten A bis D zu den entsprechenden Mischvorrichtungen mit zugehörigen Pumpen E, E', E", E'" und Ventilen, Verbindungsleitungen für die Abführung von Komponenten, Thermostaten 4, 4' und 4" zur Temperierung der Mischvorrichtungen 1, 1' und 1", eine Steuerungseinrichtung (in Fig. 7 nicht dargestellt) , welche alle Prozessstufen überwacht und regelt, eine Anzeigeeinrichtung (in Fig. 7 nicht dargestellt) mit einem Bedienteil zur Visualisierung und Eingabe von Prozessvariablen.

Die Verbindungsleitungen zwischen den Mischvorrichtungen 1 und 1' sowie 1' und 1" sind mit Temperaturfühlern T zur Temperaturführung der Mischkammern ausgestattet.

Die Mischvorrichtungen sowie Verbindungsleitungen weisen Sensoren zur Produkt und Prozesskontrolle auf (in Fig. 7 nicht dargestellt).

Weiterhin können die Auslaufleitungen der einzelnen Mischvorrichtungen weitere Sensoren aufweisen, die z. B. eine kontinuierliche Partikelgrößenmessung, direkt oder im Bypass, eine Temperaturmessung, eine Druckmessung oder ähnliches ermöglichen.

Anhand eines Emulgierbeispiels zur Herstellung eines Shampoos wird die Anlage nach Fig. 7 erläutert.

Folgende Komponenten werden in den Vorratstanks gelagert:

| | |
|---|---|
| Komponente A: | Sodium Laureth Sulfate (SLES) 70 % |
| Komponente B: | Wasser, Konservierungsmittel, Co-Tensid |
| Komponente C: | Perlglanzmittel |
| Komponente D: | Wasser, Salz, Farbstoffe |

Die Kernbestandteile bilden die drei Mischvorrichtungen 1, 1', 1" welche jeweils mit einem Thermostatmantel ausgestattet sind und über einen eigenen Heiz-/Kühlkreislauf verfügen. In der Mischvorrichtung 1 wird eine hochviskose Gelphase aus den Einzelkomponenten (Komponente A, Komponente B, Komponente C) erzeugt. Die Mischvorrichtung 1' dient zum Nachrühren der Gelphase welche dann in die Mischvorrichtung 1" geleitet, um dort mit Komponente D verdünnt zu werden.

Komponente A, Komponente B und Komponente C werden mit Exzenterschneckenpumpen E, E' und E" angesaugt und im Verhältnis 1:3,71:0,36 der ersten Mischvorrichtung 1' zugeführt. Die Komponente D wird der Mischvorrichtung 1" mit die Pumpe E'" im Verhältnis 2,21 bezogen auf Komponente A zugeführt. Die Pumpen wurden so ausgewählt, dass sie einen gleichmäßigen, nicht pulsierenden Komponentenstrom liefern. Dabei mussjede Pumpe einen minimalen stabilen Förderstrom liefern, der für eine Gesamtproduktionsmenge von 100 kg bis 300 kg pro Stunde ausreichend ist. Exzenterschneckenpumpen eignen sich in dem gezeigten Schema sehr gut, da sie unkritisch in Bezug auf wechselnde Viskositäten sind.

Aufgrund der Tatsache, dass in der in Fig. 7 schematisch dargestellten Anlage keine Durchflussmesser für die einzelnen Produktströme vorhanden sind, ist es von Vorteil eine Pumpe zu wählen, welche eine lineare Förderkennlinie aufweist. Somit können wechselnde Förderraten einfach errechnet werden. In Systemen mit Durchflussmessern (Volumen oder Masse) können auch nichtlineare Pumpen wie z. B. Zahnradpumpen problemlos eingesetzt werden.

Die Pumpen E sind für einen Gegendruck bis 5 Bar ausgelegt. Über die Ausgänge Komponente A bis Komponente D kann einfach die Fördermenge der jeweiligen Pumpe bei einer eingestellten Umdrehungszahl bestimmt werden. Hier bietet sich die Bestimmung der Fördermenge bei 100 U/min an. Der entsprechende Förderstrom wird in ein vorher austariertes Gefäß für die Dauer von einer Minute aufgefangen und gewogen. Dieser Vorgang wird dreimal wiederholt und aus allen drei Förderströmen der Mittelwert gebildet. Der so gemittelte Förderstrom der Pumpe kann über den Dreisatz dann auf den gewünschten, für die Rezeptur benötigten Förderstrom umgerechnet werden.

Mit den so ermittelten Drehzahlen werden nun die Pumpen und die Motoren der Rührorgane gestartet. Die Pumpen fördern nun die benötigten Mengen der einzelnen Komponenten in die Mischvorrichtungen, um das Endprodukt zu erhalten. Über die eingebauten Drucksensoren P kann der entstehende Druck kontrolliert werden, und bei Überdruck in der Rohrleitung oder den Mischvorrichtungen kann die Steuerung entsprechend reagieren und eine Warnung ausgeben, die Anlage stoppen, oder ähnliche Gegenmaßnahmen ergreifen. Durch die in den Auslaufleitungen der einzelnen Mischvorrichtungen integrierten Temperaturfühler kann die Produkttemperatur erfasst werden und zum Steuern der Temperiergeräte des Doppelmantels genutzt werden oder anderweitig in der Steuerung oder einem Peripheriegerät verarbeitet werden.

Bei der Herstellung des Shampoos wurde die Gesamtleistung der kompletten Anlage in Abhängigkeit vom Gesamtdurchfluss gemessen.

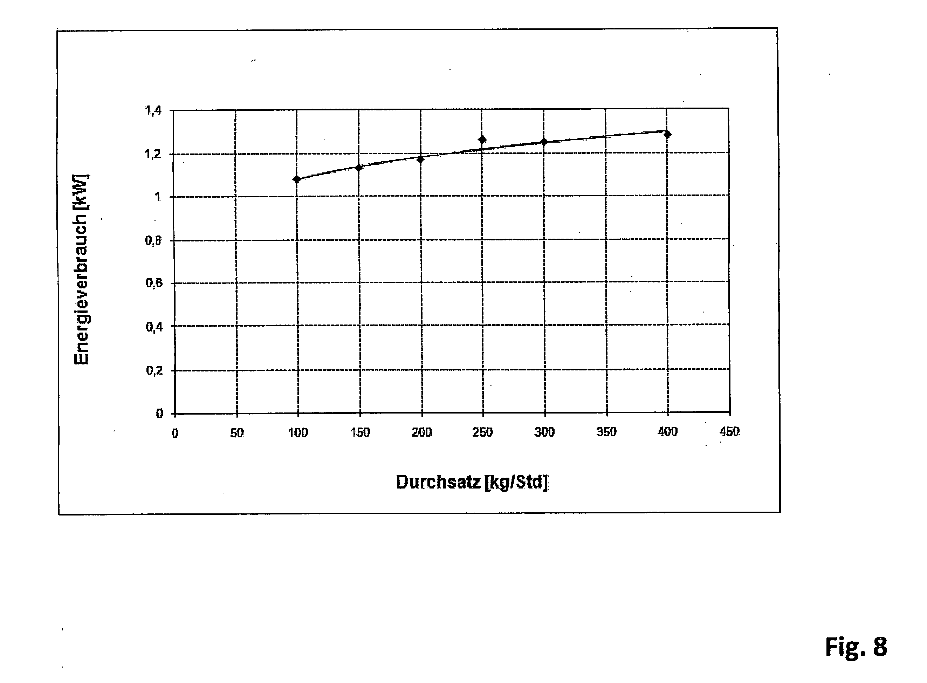

Gemessen wurde die Gesamtleistungsaufnahme bei einem Durchsatz von 100 kg/Std, 150 kg/Std, 200 kg/Std. 250 kg/Std, 300 kg/Std. und 400 kg/Std, Die ermittelten Messwerte wurden in einem XY-Diagramm aufgetragen **(****Fig. 8****).**

### Bedingungen:

Emulgieranlage mit 3 Mischkammern
Kammmerdurchmesser: 50 mm
Rührwerkzeug: Teildrahtrührer

### Gemessene Werte:

| Durchsatz [kg/Std] | Energieverbrauch [kW] |
|---|---|
| 100 | 1,08 |
| 150 | 1,13 |
| 200 | 1,17 |
| 250 | 1,26 |
| 300 | 1,25 |
| 400 | 1,28 |

Wenn man die Werte mit Hilfe eines Statistikprogramms extrapoliert wird selbst bei einem Durchsatz von 10000 kg/Std ein Gesamtenergiebedarf von 2 kW nicht überschritten.

## Patentansprüche

1. Emulgiereinrichtung zur kontinuierlichen Herstellung von Emulsionen und/oder Dispersionen mit
a) mindestens einer Mischvorrichtung (1), umfassend
eine allseitig luftdicht geschlossene rotationssymmetrische Kammer (2), mindestens eine Zulaufleitung (5, 6) zum Eintrag von fließfähigen Komponenten, mindestens eine Ablaufleitung (7) zum Austrag der gemischten fließfähigen Komponenten,
einem, einen laminaren Fluss gewährleistenden Rührorgan (11) mit auf einer Rührwelle (10) befestigten Rührelementen, dessen Drehachse entlang der Symmetrieachse der Kammer verläuft und dessen Rührwelle (10) wenigstens einseitig geführt ist, wobei die mindestens eine Zulaufleitung (5, 6) vor oder unterhalb der mindestens einen Ablaufleitung (7) angeordnet ist,
b) wenigstens einem Antrieb (12) für das Rührorgan und
c) mindestens einer Fördereinrichtung je Komponente oder je Komponentenmischung, **dadurch gekennzeichnet, dass**
das Verhältnis zwischen dem Abstand zwischen Zulauf- (5, 6) und Ablaufleitung (7) und dem Durchmesser der Kammer (2) > 2:1 beträgt,
wobei das Verhältnis zwischen dem Abstand zwischen Zulauf- (5, 6) und Ablaufleitung (7) und der Länge der Rührarme der Rührelemente 3:1 - 50:1 beträgt, und wobei das Verhältnis vom Durchmesser der Rührwelle (10) bezogen auf den Innendurchmesser der Kammer (2) das 0.25 bis 0.75 fache des Innendurchmessers der Kammer (2) beträgt, so dass die in die Mischvorrichtung (1) über die mindestens eine Zulaufleitung (5, 6) eingebrachten Komponenten rührbar und kontinuierlich über einen zulaufseitigen turbulenten Mischungsbereich, in dem die Komponenten durch die durch die Rührorgane (11) ausgeübten Scherkräfte turbulent vermischbar sind, einen sich daran anschließenden perkulierenden Mischungsbereich, in dem die Komponenten weiter durchmischbar sind und die turbulente Strömung abnimmt, einen ablaufseitigen laminaren Mischungsbereich, in dem eine lyotrope, flüssigkristalline Phase in der Mischung der Komponenten einstellbar ist, in Richtung der Ablaufleitung (7) transportierbar sind.

2. Emulgiereinrichtung nach Anspruch 1, **dadurch gekennzeichnet, dass** die Kammer (2) die Form eines Hohlzylinders, eines Kegelstumpfs, eines Trichters, eines Kuppelstumpfes oder eine aus diesen geometrischen Formen zusammengesetzte Form aufweist, wobei der Durchmesser der Kammer von der Zulaufleitung (5, 6) zur Ablaufleitung (7) gleich bleibt oder abnimmt und das Rührorgan (11) der Form der Kammer (2) korrespondierend angepasst ist.

3. Emulgiereinrichtung nach Anspruch 1, **dadurch gekennzeichnet, dass** das Verhältnis zwischen dem Durchmesser der Kammer (2) und dem Abstand zwischen Zulauf-(5, 6) und Ablaufleitung (7) im Bereich von 1:50 bis 1:2 liegt.

4. Emulgiereinrichtung nach Anspruch 1, **dadurch gekennzeichnet, dass** das Verhältnis des Durchmessers der Rührwelle (10) bezogen auf den Innendurchmesser der Kammer (2) dK vorzugsweise im Bereich 0.3 - 0.7 *dK liegt.

5. Emulgiereinrichtung nach Anspruch 1, **dadurch gekennzeichnet, dass** wenigstens ein Bestandteil der Rührelemente parallel und beabstandet zur Innenwandung der Kammer (2) angeordnet ist.

6. Emulgiereinrichtung nach Anspruch 1, **dadurch gekennzeichnet, dass** das Rührorgan (11) ein Vollblatt- oder Teilblattrührer oder ein Volldrahtrührer oder ein Teildrahtrührer oder eine Kombination dieser ist.

7. Emulgiereinrichtung nach einem der Ansprüche 1 bis 6, **dadurch gekennzeichnet, dass** die Kammer (2) wenigstens einen, einen laminaren Fluss begünstigenden Strömungsbrecher (16) aufweist.

8. Emulgiereinrichtung nach einem der Ansprüche 1 bis 6, **dadurch gekennzeichnet, dass** die mindestens eine Mischvorrichtung (1) mehrere hintereinandergeschaltete, rotationssymmetrische Kammern (2, 2') aufweist.

9. Emulgiereinrichtung nach Anspruch 1, **dadurch gekennzeichnet, dass** der Mischvorrichtung (1) als erste Mischvorrichtung wenigstens eine weitere Mischvorrichtung (1') nachgeschaltet ist, wobei nach der ersten Mischvorrichtung (1) eine lyotrope und flüssigkristalline Phase in der Mischung der Komponenten vorhanden ist und die Viskosität der Mischung der wenigstens einen nachfolgenden weiteren Mischvorrichtung (1') gleich oder kleiner der Viskosität nach der ersten Mischvorrichtung (1) ist.

10. Emulgiereinrichtung nach Anspruch 1, **dadurch gekennzeichnet, dass** wenigstens ein Strömungssensor in wenigstens einer der Leitungen angeordnet ist

11. Emulgiereinrichtung nach Anspruch 1, **dadurch gekennzeichnet, dass** mindestens eine Einrichtung zur Temperierung an wenigstens eine der Leitungen gekoppelt ist, so dass die Komponenten, Komponentenmischungen und/oder Emulsionen oder Dispersionen kühl- oder heizbar sind.

12. Emulgiereinrichtung nach Anspruch 1 bis 11, **dadurch gekennzeichnet, dass** der Antrieb (12), die Fördereinrichtung und der Sensor und die Einrichtung zur Temperierung mit einer Steuerungseinrichtung für die Überwachung und Steuerung der Mischvorrichtungen (1, 1'), die Zu- und Ableitung (5, 6, 7, 7', 13) der Komponenten, Komponentenmischungen, oder Emulsionen oder Dispersionen verbunden sind, wobei die Steuereinrichtung die Anlage so steuert, dass die Viskosität der in der ersten Mischvorrichtung (1) erzeugten Mischung immer größer oder gleich der Viskosität in der (den) nachfolgenden Mischvorrichtung(en) (1') und eine laminare Strömung der durchmischten Komponenten gewährleistet ist.

13. Emulgiereinrichtung nach Anspruch 12, **dadurch gekennzeichnet, dass** die Steuereinrichtung mit einem Femwartungsmodul und/oder einem Rezeptverwaltungsmodul verbunden oder verbindbar ist.

## Claims

1. Emulsifying device for continuous production of emulsions and/or dispersions comprising
a) at least one mixing apparatus (1) comprising
- a rotationally symmetric chamber (2) sealed airtight on all sides,
- at least one inlet line (5,6) for introduction of free-flowing components,
- at least one outlet line (7) for discharge of the mixed free-flowing components,
- a stirrer unit (11) which ensures laminar flow and comprises stirred elements secured on a stirred shaft (10), the axis of rotation of which runs along the axis of symmetry of the chamber and the stirred shaft (10) of which is guided on at least one side, wherein the at least one inlet line (5,6) is arranged upstream of or bellow the at least one outlet line (7),
b) at least one drive (12) for the stirrer unit and
c) at least one conveying device per component or per component mixture, **characterized in that** the ratio between the distance between inlet (5,6) and outlet (7) lines and the diameter of the chamber (2) is ≥ 2 : 1,
wherein the ratio between the distance between inlet (5,6) and outlet (7) lines and the length of the stirrer arms of the stirrer elements is 3:1-50:1,
and wherein the ratio of the diameter of the stirred shaft (10), based on the interval diameter of the chamber (2), is 0.25 to 0.75 times the interval diameter of the chamber (2), such that the components introduced into the mixing apparatus (1) via the at least one inlet line (5,6) can be stirred and continuously transported by means of a turbulent mixing area on the inlet side, in which the components can be mixed turbulently by the shear forces exerted by the stirrer units (11),
a downstream percolating mixing area in which the components can be mixed further and the turbulent flow decreases,
a laminar mixing area on the outlet side, in which a lyotropic, liquid-crystalline phase can be established in the mixture of the components, in the direction of the outlet line (7).

2. Emulsifying device according to Claim 1, **characterized in that** the chamber (2) has the shape of a hollow cylinder, of a frustocone, of a funnel, of a frustodome, or a shape composed of these geometric shapes, the diameter of the chamber remaining constant or decreasing from the inlet line (5,6) to the outlet line (7) and the stirrer unit (11) being adapted correspondingly to the shape of the chamber (2).

3. Emulsifying device according to Claim 1, **characterised in that** the ratio between the diameter of the chamber (2) and the distance between inlet (5, 6) and outlet (7) lines is in the range from 1:50 to 1:2.

4. Emulsifying device according to Claim 1, **characterised in that** the ratio of the diameter of the stirrer shaft (10) based on the internal diameter of the chamber (2) dK is preferably in the range of 0.3 - 0.7*dK.

5. Emulsifying device according to Claim 1, **characterized in that** at least one constituent of the stirrer elements is arranged in parallel and spaced apart from the inner wall of the chamber (2).

6. Emulsifying device according to Claim 1, **characterized in that** the stirrer unit (11) is a full-blade or part-blade stirrer or a full-wire stirrer or a part-wire stirred or a combination of these.

7. Emulsifying device according to any of Claims 1 to 6, **characterized in that** the chamber (2) has at least one baffle (16) which promotes a laminar flow.

8. Emulsifying device according to any of Claims 1 to 6, **characterized in that** the at least one mixing apparatus (1) has a plurality of rotationally symmetric chambers (2, 2') connected in series.

9. Emulsifying device according to Claim 1, **characterized in that** the mixing apparatus (1) as the first mixing apparatus has at least one further mixing apparatus (1') connected downstream, a lyotropic and liquid-crystalline phase being present in the mixture of the components downstream of the first mixing apparatus (1), and the viscosity of the mixture in the at least one further mixing apparatus (1') downstream being equal to or less than the viscosity downstream of the first mixing apparatus (1).

10. Emulsifying device according to Claim 1, **characterized in that** at least one flow sensor is arranged in at least one of the lines.

11. Emulsifying device according to Claim 1, **characterized in that** at least one device for temperature control is coupled to at least one of the lines, such that the components, component mixtures and/or emulsions or dispersions are coolable or heatable.

12. Emulsifying device according to Claims 1 to 11, **characterized in that** the drive (12), the convening device and the sensor, and the device for temperature control are connected to a control device for the monitoring and control of the mixing apparatuses (1, 1'), the supply and removal (5, 6, 7, 7', 13) of the components, component mixtures, or emulsions or dispersions, the control device controlling the system such that the viscosity of the mixture obtained in the first mixing apparatus (1) is always greater than or equal to the viscosity in the downstream mixing apparatus(es) (1') and a laminar flow of the mixed components is ensured.

13. Emulsifying device according to Claim 12 **characterized in that** the control device is or can be connected to a remote maintenance module and/or a formula management module.

## Revendications

1. Dispositif d'émulsification permettant de fabriquer en continu des émulsions et/ou des dispersions et présentant :
a) au moins un dispositif de mélange (1) qui comporte une chambre (2) à symétrie de rotation, fermée de manière étanche à l'air par tous ses côtés,
au moins un conduit d'amenée (5, 6) qui permet d'introduire des composants fluides, au moins un conduit d'extraction (7) qui permet d'extraire les composants fluides mélangées,
un organe de brassage (11) assurant un écoulement luminaire et présentant des éléments de brassage fixés sur un arbre de brassage (10) dont l'axe de rotation s'étend le long de l'axe de symétrie de la chambre et dont l'arbre de brassage (10) est guidé sur au moins une côté, le ou les conduits d'amenée (5, 6) étant disposés en avant ou en dessous du ou des conduits d'extraction (7),
b) au moins un entraînement (12) pour l'organe de brassage et
c) au moins un dispositif de transport pour chaque composant ou pour chaque mélange de composant, **caractérisé en ce que**
le rapport entre la distance qui sépare le conduit d'amenée (5, 6) et le conduit d'extraction (7) et le diamètre de la chambre (2) est > 2:1,
**en ce que** le rapport entre la distance qui sépare le conduit d'amenée (5, 6) et le conduit d'extraction (7) et la longueur des bras de brassage des éléments de brassage vaut de 3:1 à 50:1,
**en ce que** le rapport entre le diamètre de l'arbre de brassage (10) et le diamètre intérieur de la chambre (2) représente de 0,25 à 0,75 fois le diamètre intérieur de la chambre (2) de telle sorte que les composants apportés dans le dispositif de mélange (1) par le ou les conduits d'amenée (5, 6) peuvent être brassés et transportés en direction du conduit d'extraction (7) de manière continue par l'intermédiaire d'une zone turbulente de mélange située côté amenée, dans laquelle les composants peuvent être mélangées en conditions turbulentes par les forces de cisaillement exercées par les organes de brassage (11), suivie par une zone de mélange à percolation dans laquelle les composants peuvent être mélangés plus intimement et l'écoulement turbulent diminue et par une zone laminaire de mélange située côté extraction et dans laquelle une phase lyotrope de cristaux liquides peut s'établir dans le mélange des composants .

2. Dispositif d'émulsification selon la revendication 1, **caractérisé en ce que** la chambre (2) présente la forme d'un cylindre creux, d'un tronc de cône, d'un entonnoir, d'un tronc de sphère ou d'une forme constituée de l'assemblage de ces formes géométriques, le diamètre de la chambre restant identique ou diminuant entre le conduit d'amenée (5, 6) et le conduit d'extraction (7) et l'organe de brassage (11) ayant une forme adaptée à la forme de la chambre (2).

3. Dispositif d'émulsification selon la revendication 1, **caractérisé en ce que** le rapport entre le diamètre de la chambre (2) et la distance entre le conduit d'amenée (5, 6) et le conduit d'extraction (7) est de l'ordre de 1:50 à 1:2.

4. Dispositif d'émulsification selon la revendication 1, **caractérisé en ce que** le rapport entre le diamètre de l'arbre de brassage (10) et le diamètre inférieur de la chambre (2) dK est de préférence situé dans la plage de 0,3 à 0,7*dK.

5. Dispositif d'émulsification selon la revendication 1, **caractérisé en ce qu'**au moins un composant des éléments de brassage est disposé parallèlement et à distance de la paroi intérieure de la chambre (2).

6. Dispositif d'émulsification selon la revendication 1, **caractérisé en ce que** l'organe de brassage (11) est un brasseur à pales pleines ou à pales partielles, un brasseur à fils pleins, un brasseur à fils partiels ou une combinaison d'entre eux.

7. Dispositif d'émulsification selon l'une des revendications 1 à 6, **caractérisé en ce que** la chambre (2) présente au moins un dispositif (16) de rupture d'écoulement qui favorise un écoulement luminaire.

8. Dispositif d'émulsification selon l'une des revendications 1 à 6, **caractérisé en ce que** le ou les dispositifs de mélange (1) présentent plusieurs chambres (2, 2') raccordées les unes à la suite des autres et présentant une symétrie de rotation.

9. Dispositif d'émulsification selon la revendication 1, **caractérisé en ce que** le dispositif de mélange (1) utilisé comme premier dispositif de mélange est suivi par au moins un, autre dispositif de mélange (1'), une phase lyotrope et à cristaux liquides étant présente dans le mélange des composants après le premier dispositif de mélange (1) et la viscosité du mélange dans le ou les dispositifs de mélange (1') qui suivent est égale ou inférieure à la viscosité qui règne après le premier dispositif de mélange (1).

10. Dispositif d'émulsification selon la revendication 1, **caractérisé en ce qu'**au moins un capteur d'écoulement est disposé dans au moins l'un des conduits.

11. Dispositif d'émulsification selon la revendication 1, **caractérisé en ce qu'**au moins un dispositif de maintien à température est raccordé à au moins l'un des conduits de telle sorte que les composants, les mélanges de composants et/ou les émulsions ou dispersions puissent être refroidis ou chauffés.

12. Dispositif d'émulsification selon les revendications 1 à 11, **caractérisé en ce que** l'entraînement (12), le dispositif de transport et le capteur et le dispositif de maintien à température sont raccordés à un dispositif de commande qui assure la surveillance et la commande des dispositifs de mélange (1, 1'), de l'amenée et de l'extraction (5, 6, 7, 7', 13) des composants, des mélanges de composants, des émulsions ou des dispersions, le dispositif de commande cammandant l'installation de telle sorte que la viscosité du mélange formé dans le premier dispositif de mélange (1) soit de manière garantie toujours supérieure ou égale à la viscosité qui règne dans le ou les dispositifs de mélange (1') qui suivent et de manière à garantir un écoulement laminaire des composants mélangés intimement.

13. Dispositif d'émulsification selon la revendication 12, **caractérisé en ce que** le dispositif de commande est relié ou peut être relié à un module d'entretien à distance et/ou à un module de gestion de formulations.
